# EUROPEAN PATENT APPLICATION

(11) **EP 3 766 981 A1**
(43) Date of publication of application: **20.01.2021**
(21) Application number: 19305950.8
(22) Date of filing: 18.07.2019
(51) Int. Cl.: C12N 15/87, A61K 48/00, C08L 71/00

(54) **CATIONIC POLOXAMERS AND THEIR USE IN TRANSDUCTION**

(71) Applicant: OZ Biosciences, 13288 Marseille Cedex 9 (FR)
(72) Inventor: POULHES, Florent, 13013 MARSEILLE (FR); SAPET, Cédric, 13360 ROQUEVAIRE (FR); ZELPHATI, Olivier, 13830 ROQUEFORT-LA-BEDOULE (FR)
(74) Representative: Santarelli

(57) **Abstract**

The invention relates to a method for the enhancement of the transduction of a target cells by a viral vector using a cationic block-copolymer introduced as an additive alone or formulated with nanoparticles. The method comprises a step of contacting a target cells with viruses and a cationic block co-polymer. The structure of this additive incorporates both hydrophilic and hydrophobic regions which represents different areas in the backbone of the polymer. This polymeric construction is ended by cationic chemical functions which contribute to further enhance the viral transduction. The invention also relates to new cationic poloxamers that can be used in the method of the invention. Furthermore, another embodiment of the present invention relates to the colloidal stabilization of iron-based nanoparticles using these polymers and their use in increasing transduction efficiency.

## Description

The method described in the present document relates to the enhancement of the transduction of a target cells by a viral vector using a cationic block-copolymer introduced as an additive alone or formulated with nanoparticles (also named in the text chemical adjuvant). The method comprises a step of contacting a target cells with viruses and a cationic block co-polymer. The structure of this additive incorporates both hydrophilic and hydrophobic regions which represents different areas in the backbone of the polymer. This polymeric construction is ended by cationic chemical functions which contribute to further enhance the viral transduction. The invention also relates to new cationic poloxamers that can be used in the method of the invention. Furthermore, another embodiment of the present invention relates to the colloidal stabilization of iron-based nanoparticles using these polymers and their use in increasing transduction efficiency.

In gene and cell therapy applications, the use of viruses is the method of choice for genetic modifications of most cells especially primary cells. This kind of vectors, especially retroviruses, allows stable gene expression in infected cells and became quickly key cornerstones for both research and therapeutic applications.

In recent years, Lentiviral (LV) vectors have been used successfully to infect human or other mammalian cells, either proliferating or not. The success of these procedures requires in most cases the use of LV pseudotyped with glycoproteins. Such vectors can thus integrate stably into the chromosomes of the target cell. In the same way, Adenovirus and adeno-associated virus have been extensively used as well for genetic modification or engineering of a variety of cell and tissues.

Currently there is a great interest in finding highly efficient transduction conditions involving lentiviruses or adenovirus or adeno-associated virus, or more generally viruses, to deliver gene into therapeutic cells, including but not limited to lymphocytes, immune cells, lymphoid cell lines, stem cells and primary hematopoietic stem cells. Recent clinical advances are based on the transfer of genetic material into primary lymphocytes, hematopoietic tumor cells or pluripotent stem cells which is known to be difficult to accomplish. Working on such cells often implies the use of highly concentrated and high purity grade virus preparation to be fully efficient. As a consequence, large-scale transduction of patient cells requires upscaling of virus production and elaborated processing to obtain high viral titre and concentrated virus stock. Such optimizations imply thus an important financial cost.

It is of a primary importance to improve the efficiency of viral transductions in order to reduce the needed virus production volume, and as a consequence the cost of clinical trials. Furthermore, working with lower multiplicity of infection (MOI) would reduce the risk of mutagenesis upon insertion of multiple virus copies per patient's cell.

In the present text, the expression "such as" should be understood as being nonlimiting and should be understood as "such as an example" which implies that the elements listed after it are only examples cited among others not mentioned that can perfectly fit within the scope of the invention.

Several strategies have been used to improve the efficiency of a transduction procedure involving viruses.

The first improvement point relies on the vector itself. The purity and quality of the viral preparation, the multiplicity of infection (MOI) (ratio of the number of infecting agent - *e.g. infectious* viral particles- to the number of host *-e.g.* cells) and the virus construction are critical parameters to control in order to reach optimal efficiency of the transduction. Indeed, some studies report that the presence of endogenous inhibitors in virus may explain the decrease of efficiency in transduction procedures. The use of concentrated and purified virus preparations that may help to circumvent these concerns, have been obtained in the past by ultracentrifugation, or ultrafiltration strategies. Efficient transduction procedures require also optimal cell density before virus application, limited cell passage number and careful control of the cell culture medium composition.

Besides the problem related to the purity and quality of the viral vector, there exist some basic biophysical constraints which limit the efficiency of viral gene transfer. Several groups have indeed demonstrated that the slow diffusion and rapid inactivation of viruses are major contributors to the relatively low observed efficiencies. With virus particles able to diffuse only a few microns before losing bioactivity, a large proportion of the initially active virus particles become inactive before they can interact with a target cell, fundamentally limiting achievable levels of transduction efficiency.

Mechanical or physical approaches allowed to enhance the number of viral particles still infectious while reaching the target cells. Among other, centrifugation (or spinoculation) techniques (A.B. Bahnson et al.; J. Virol. Methods 1995, 54: 131-143), flow-through transduction (S. Chuck et al.; Hum. Gene Ther.1996, 7: 743-750), or the use of magnetic nanoparticles (MNPs) (Sloutskin A. et al.; J Virol Methods. 2014, 206:128-32) can be cited as able to increase the concentration of active viral particles onto the cell's surface. Those techniques have in common to increase the likelihood and frequency of active virus-cell interactions by adding a convective component to the mass transport of virus. Magnetofection™ technology that used principally cationic magnetic nanoparticles have been able to promote, enhance and synchronize virus infection and adsorption in a large variety of biological models together *in vitro, ex-vivo* and *in vivo* (C. Plank, et al.; Adv Drug Del Rev 2011, 63: 1300-1331). While having proved to be efficient, their expense and difficult scale-up somehow limit their potential as component in a large-scale *in-vivo* clinical setting.

Another strategy relied on the use of chemical adjuvants. A wide number of chemical adjuvants have been used over past years to facilitate the transduction such as recombinant polypeptides, polycationic compounds, or neutral compounds such as poloxamers. Among all the molecules tested, polycations such as cationic liposomes or cationic polymers such as, polyethylenimine (PEI), ^{,} DEAE-dextran, protamine sulfate or poly-L-lysine have been widely investigated.

However, the most common cationic additive one can find in described transduction procedures is called polybrene. It is a non-protic cationic and linear polymer which demonstrated to improve significantly gene transduction rates in a large range of target cells. These interesting features secured the polybrene's leading position in the field of adjuvant-assisted viral gene delivery process. Unfortunately, polybrene shares with many polycationic molecules also used for this purpose, negative features which limited its impact in clinical trials. First and foremost, polycations with high positive charge density, are known to be somehow toxic for the target cells, excluding their use on sensitive cell type, such as primary patient cells. Consequently, polybrene can only be used in short application times and in low concentrations (under 10 µg/mL) to limit toxicity on target cells. This aspect is clearly a major drawback for the use of polybrene in clinical applications, especially when the targets are highly sensitive cells, such as primary hematopoietic stem cells.

Beside polycations, another class of compounds used as transduction enhancers are linear or X-shaped non-ionic poloxamers (A.V. Kabanov et al.; J. Control. Release, 2002, 82:189-212; A. Rey-Rico et al.; Int. J. Mol. Sci. 2018, 19:775). Poloxamer refers to well-known class of non-ionic triblock copolymers composed by a central hydrophobic chain of polyoxypropylene (PPO) surrounded by two hydrophilic chains belonging to the polyethylene oxide (PEO) family [PEO-PPO-PEO]. These poloxamers can be linear or X-shaped, this latest being known as poloxamines. Included in this family are also the meroxapols, also known as "reverse poloxamers" in the literature, which present a hydrophilic block based on a polyethylene oxide polymer, surrounded by two hydrophobic blocks, based on polypropylene oxide polymers. By extension we will consider in this invention their X-shaped variation, the "reverse poloxamine". All those polymers (poloxamers, poloxamine, meroxapols, reverse poloxamine) will be referred as poloxamers in the rest of the document.

Poloxamers can also be named according to their trade name, which might be "Pluronic" or "Symperonic", depending on the manufacturer. These molecules are characterized by different hydrophilic/lipophilic (*e.g*. hydrophobic) balances (HLB) and critical micelles concentrations (CMC). These structural features have shown to play a critical role in their biological properties (A; Kabanov A. et al.; Adv. Gen. 2005, 53: 231-61). Besides their well-described self-assembly and micellization behaviours, poloxamers have been largely used in cosmetics, galenic and diverse formulations. They also have been used in non-viral gene delivery procedures, for *in vitro* and or *in vivo* applications. as well as for electroporation due to their sealing agents' properties. They also have been used in combination with cationic polymers to form polyplexes with DNA.

Poloxamers have been shown to act as polymeric excipients during virus-mediated gene delivery procedures (AV. Kabanov et al.; Adv Drug Del Rev 2002, 54:223-3). The first example of such utilization has been reported by March et al who used poloxamer 407 (known also as F127) as a rate and frequency transduction enhancer in vascular smooth muscle cells using recombinant adenovirus vector (KL. March, et al.; Hum. Gene Ther, 1995, 6:41-53). This study emphasized on the modulation of the pharmacokinetics of adenoviral mediated gene delivery by the poloxamer. While the mechanism underlying this positive effect is not explicitly understood in the report, the authors hypothesized that Poloxamer 407 formed a gel under the experimental conditions of the experiment, which served as "delivery reservoir" for the virus improving gene delivery by maintaining elevated pericellular concentrations of the vector.

Following this pioneering work, several other studies have highlighted the positive effect of poloxamer during transduction experiments. Among other the use of poloxamer 407 in arterial transduction in rats using adenoviruses as vectors (LJ. Feldman, et al.; Gene Ther. 1997, 4, 189-198) or the use of poloxamer 407 as gels for percutaneous adenovirus mediated gene transfer in conjunction with stent implantation in rabbit iliac arteries can be cited (Van Belle et al. Hum. Gene Ther. 1998, 9, 1013-1024). The enhancing behaviours of poloxamer in transduction have also been demonstrated using lentivirus as vectors in several studies notably with endothelial or smooth muscle cells as targets (KL. Dishart, et al. J. Mol. Cell. Cardiol. 2003, 35:739-748) In this case, AAV serotypes and vesicular stomatitis virus glycoprotein-pseudotyped lentiviral system have been put in parallel with similar results, enhanced by the presence of poloxamer 407 as adjuvant. Another really interesting study involves the use of AAV associated with micelles of linear (Pluronic F68) or X-shaped (poloxamine Tetronic T908) poloxamers. In this case, micelles encapsulation using poloxamers allowed an increase in both the stability and the bioactivity of the AAV, leading to higher level of transgene expression both *in vitro* and in experimental osteochondral defects without detrimental effects on the cells.

Since then, enthusiasm for the use of poloxamer as adjuvant in virus-mediated gene delivery procedures has proved consistent. The perspectives emerging those last years concerning new gene therapy clinical trials involving lentivirus, strengthened and extended this interest and gave rise to several patent application involving poloxamers in the context of virus-mediated gene delivery aimed toward both *in vitro* and *in vivo* applications. Among several others, the combined use of polybrene and poloxamer 338 in a retrovirus-mediated gene transfer strategy (WO2013/127964A1, WO2017/139576A1), or the derivation of primary T-cells to include CAR (chimeric antigenic receptor) using lentivirus and a wide range of adjuvants which includes various poloxamers (WO2015/162211A1, WO2018/148502A1) can be cited.

However, the efficiencies of poloxamers are highly dependent of the type of cells targeted, of the poloxamer structure used and requires in many cases the addition of a cationic polymer such as polybrene. Moreover, their non-ionic property is unfavourable to their combination or formulation with nanoparticles such as iron-based magnetic nanoparticles. Indeed, magnetic nanoparticles have been shown to increase and accelerate viral transduction efficiency in a variety of model. Consequently, it could have been envisioned that addition of magnetic nanoparticles and poloxamer lead a powerful synergistic effect. Unfortunately, the non-ionic nature of poloxamer lead to nanoparticles destabilization or precipitation which hampered their use as coating or formulating agent of nanoparticles.

Due to their interesting properties, several studies have focused on the chemical modifications of poloxamers. The objective was to add new physico-chemical properties to the existing unique features of these polymers in order to circumvent certain limitations.

For instance, in a non-viral strategy and in order to allow the poloxamer to interact and condense naked plasmid DNA, several groups used poloxamer's hydroxyl functions as attaching points to link covalently cationic polymers of different natures. For instance, it was described the behaviour as non-viral vector of a block copolymer composed of Pluronic 123 attached to a branched polyethyleneimine with a molecular weight around 2000 Da. The same concept has been also applied to poloxamer 407 linked covalently with polylysine, or the development of new non-viral vectors based on acrylate-modified poloxamers condensed with polyethyleneimine for efficient gene delivery. In the majority of these cases, the modification took place at the hydroxyl end group, (Gyulain G. et al eXPRESS Polymer Letters 2016, 10: 216-26). The reactions involved in these procedures often relied on the polyethylene glycol chemistry.

However, none of such modifications with cationic groups or polymers linked at the extremity of poloxamer has been realized and attempted in the context of enhancing viral transduction to circumvent the requirement of using an additional cationic polymer such as polybrene for increasing their efficiency in several types of cells as discussed before. In the same way, the potential synergistic effect of the modified poloxamer with magnetic nanoparticles have not been apprehended and monitored. The present invention intends to solve the technical problems related to poloxamers used for enhancing viral transduction alone or in combination with magnetic nanoparticles described here above. The invention also intends to create an alternative and/or improved means and methods for transducing cells with viruses.

Furthermore, this invention also relates to the modification of the poloxamers ending groups to be able to modulate further the characteristics and the behaviour of the chemical adjuvant. More precisely, the invention describes the systematic introduction of cationic organic functions at the end of the polymeric backbone of poloxamer to create in one entity the synergistic properties to enhance the viral transduction instead of two separate entities (non-ionic and ionic) and to be able to be formulated with magnetic nanoparticles as transduction enhancers. The added value of the present invention described in the present document is to associate synergistically the surfactant properties of poloxamers with cationic moieties able to interact with both virus and cells, into the same polymeric entity. Whereas the mixing of a poloxamer and a cationic polymer has already been described, this is the first time from Applicant knowledge that a single polymer presenting both an amphiphilic character and cationic functions is used in conjunction with a virus.

More precisely, the present invention describes the systematic introduction of cationic organic functions at the end of the polymeric backbone of poloxamer.

The present invention describes for the first time a method that uses cationic poloxamers to enhance viral transduction of target cells.

The invention described in the present document relates to a method to enhance the transduction of a target cells by a viral vector using a cationic poloxamer (otherwise that may be named in this text "chemical adjuvant" or "additive") introduced as an additive alone or formulated with nanoparticles.

According to the invention, the method comprises a step of contacting a target cells with viruses and a cationic poloxamer. The structure of this additive incorporates both hydrophilic and hydrophobic regions which represents different areas in the backbone of the polymer. This polymeric construction is ended by cationic chemical functions which contribute to further enhance the viral transduction.

Furthermore, another embodiment of the present invention relates to the colloidal stabilization of iron-based nanoparticles using those cationic poloxamers and their use in increasing transduction efficiency.

The Applicant focused on the modification of several poloxamers into cationic poloxamers, that are combined with viruses and put in contact with a target cells to dramatically increase efficiency rates of transduction without any kind of toxicity. The details of this invention are described in the following claims, while the technical benefits will be discussed in the embodiments related to them.

According to this the first object of the invention relates to a method of transduction of a target cells by a viral vector using a cationic poloxamer.

In the present text the term "transduction" refers to a really common and accepted meaning in the scientific community. Transducing invokes the process to introduce native, wild type or recombinant genetic material via a virus into the target cell, and optionally its integration into the genome. This process is also often referred as "infection"; both terms will be used interchangeably in this document. In contrast, transfection refers to a method of delivering genetic materials into cells with a synthetic or non-viral delivery systems. The nature of the genetic material introduced, and the mechanisms involved in this step are dependent on the type of virus used in the transduction procedure. For example, the genetic materials introduced by a virus can encode a large variety of gene of interest such as reporter genes, enzymes, endonucleases, nucleases or recombinase for genome editing, short harping RNA, antisense RNA for gene silencing, mRNA, tRNA, receptors such as engineering antigen receptor (T cell receptor, chimeric antigen receptor, chimeric cytokine receptor...), growth factors, hormone, cell surface proteins, secreted proteins, signalling proteins or any polypeptides or proteins or nucleic acids with therapeutic interest.

Precisely the invention relates to a method of transduction comprising at least one step of contacting a target cell with a viral vector and a cationic poloxamer.

According to the invention the claimed method of transduction can be performed with known cationic poloxamers as well as new cationic poloxamers as for example those newly synthetized by the Applicant and also claimed below in the present document.

According to the invention any known virus that can be used as a viral vector (native, recombinant, pseudo-type or wild type) and that can be used in transduction can be used in the method according to the invention.

By way of example and without limitation of the invention, viruses (viral vector) for use in practicing the invention include
a) double stranded DNA viruses such as for example adenovirus, adeno-associated virus, canine hepatitis virus, papillomavirus, polyomavirus, herpes simplex virus (HSV), Epstein Barr virus, cytomegalovirus, varicella zoster virus, smallpox virus, vaccinia virus, hepatitis B virus;
b) single stranded DNA viruses such as for example parvovirus, anellovirus;
c) double stranded RNA viruses such as for example reovirus, rotavirus;
d) single stranded RNA viruses such as for example picornavirus, coxsackie virus, calicivirus, togavirus, alphavirus, arenavirus, flavivirus, pestivirus, West Nile virus, orthomyxovirus, influenza virus, enterovirus, poliovirus, paramyxovirus, measles virus, newcastle disease virus, bunyavirus, rhabdovirus, vesicular stomatitis virus (VSV), filovirus, coronavirus, astrovirus, bornavirus, arterivirus, hepevirus;
e) reverse transcribing viruses such as for example retrovirus, lentivirus, hepatitis B virus and
f) other viruses such as for example arbovirus, bacteriophage.

Preferentially, the viral gene delivery vehicle can be any gene therapy delivery vehicle known in the art for use in gene therapy, including, but not limited to, an adenovirus, an adeno-associated virus (AAV), a lentivirus, a retrovirus, a gamma-retrovirus, a herpes virus and other viruses.

A brief description of the main viruses associated with this invention can be found below:
- Retroviruses are of the mainstays of current gene therapy approaches, mostly because of their ability to integrate into the host genome in a stable fashion. To do that, they use a retro-transcriptase converting their RNA into DNA, which is then integrated into the host genome through an integrase. Replication-defective retroviruses are the most common choice in most studies, as modification of their genome still allows them to infect target cells and to deliver their viral payload, but prevents the following pathway that leads to cell lysis and death. This family includes the gamma-retrovirus which are popular for gene therapy due to their advantages over other retroviral vectors in particular because of their genome are very simple and easy to use.
- Despite their tremendous potential as vectors, retroviruses suffer from several drawbacks such as they cannot infect cells that are not actively dividing. Furthermore, an important number of viral particles may lead to insertional mutagenesis that might causes cancer or leukemia. Retroviruses encompass a lot of different viral species that may be used in gene therapy based clinical trials, the main one being lentiviruses and gamma-retroviruses. Those two subclasses have been the subject of more than 300 clinical trials.
- Lentiviruses are a subclass of retroviruses, and have the potency to integrate their genome into host cells. Furthermore, they present the unique advantage to be equally efficient on dividing or non-dividing cells. Lentiviruses used in gene therapy are in most cases non-replicative for safety reasons. That means that they are still infective but unable to enter into any replicative cycle, lacking helper proteins to provide the missing viral proteins for the production of new virions. Most of the time, the genes necessary for the replication are replaced by an expression cassette containing a target sequence for introduction into the host's genome. This sequence may include for example, gene encoding for a therapeutically valuable protein or a marker, or a regulatory sequence for RNA interferences or miRNA expression.
- Adenoviruses possess a linear dsDNA genome and are able to replicate in the nucleus of cells using the host's replication machinery. As opposed to lentiviruses, adenoviral DNA does not integrate into the genome and is not replicated during cell division. Their primary applications are in gene therapy and vaccination. Since humans commonly come in contact with adenoviruses, which cause respiratory, gastrointestinal and eye infections, majority of patients have already developed neutralizing antibodies which can inactivate the virus before it can reach the target cell. To overcome this problem, scientists are currently investigating adenoviruses that infect different species to which humans do not have immunity.
- Adeno-associated virus (AAV) is a small virus that infects humans and some other primate species. AAV is not currently known to cause disease, and causes a very mild immune response. AAV can infect both dividing and non-dividing cells and may incorporate its genome into that of the host cell. Moreover, AAV mostly stays as episomal (replicating without incorporation into the chromosome); performing long and stable expression. These features make AAV a very attractive candidate for creating viral vectors for gene therapy. However, AAV can only bring up to 5kb which is considerably small compared to AAV's original capacity.
- Furthermore, because of its potential use as a gene therapy vector, researchers have created an altered AAV called self-complementary adeno-associated virus (scAAV). Whereas AAV packages a single strand of DNA and requires the process of second-strand synthesis; scAAV packages both strands which anneal together to form double stranded DNA. By skipping second strand synthesis scAAV allows for rapid expression in the cell. Otherwise, scAAV carries many characteristics of its AAV counterpart.

In the context of the invention, the cationic poloxamer and the viral vector have to be put in contact with the target cell for the transduction to occur. Contacting the different elements for efficient transduction has been depicted numerous times in the literature, and is deeply dependant on the nature of the chosen viral vector and of the target cell. Some cells being harder to infect than others, they may need specific care prior contact, such as transition in specific culture medium or longer contacting times with both the virus and the chemical adjuvant.

According to the invention the cationic poloxamer and the viral particle can be added at the same time onto the cells as mixture, or sequentially. However, to ensure an efficient transduction procedure, it is of a prime importance to allow the contact between the virus, the cationic poloxamer and the cells for a long enough time. Contact or incubation times covered by the scope of this invention ranges from 5 seconds to 3 months. More preferentially contact times should be comprised within 10 minutes to 2 weeks, preferentially within 20 minutes to 1 week. More preferentially it should be comprised within 0.5h to 120h.

Exemplary conditions can be found in the "example" section of this document. The cationic poloxamer and the viral particle can be added at the same time onto the cells as mixture, or sequentially. Optimized conditions for transduction following the guidelines of this invention have been described numerous times with different viral vectors, such as lentiviruses (Arnoult et al Nature 2017, 549:548-552;), AAV (Yelamarthi, T., Thesis Dissertation, 2012. University of Houston), or adenoviruses (Sapet et al Pharm Res. 2012, 29:1203-18).

Preferentially the correct quantities of virus and cationic poloxamer will be mixed together prior their deposition onto the cells. The obtained mixture requires gentle mixing before pursuing.

According to the invention a wide array of multiplicity of infection (MOI) or viral titers may be used depending on the target cells, on the chosen viral vector, and on the transduction needs. Several parameters can optionally be varied to enhance the efficiency of the transduction procedure. One skilled in the art will easily define the correct MOI in function of the used viral vector. As example, preferred MOI ranging from 0.001 to 5000000 will be considered.

According to the method of the invention, the cationic poloxamer can be provided at a stock concentration from 0.01 to 500 mg/mL, preferentially from 0.05 to 300 mg/mL, more preferentially from 1 to 200 mg/mL, even more preferentially from 5 to 150 mg/mL.

According to the method of the invention, the cationic poloxamer is used at a final concentration obtained by diluting the stock solution hereabove from 1 to 100000-fold, preferentially from 2 to 10000-fold, more preferentially from 2 to 5000-fold.

Optionally, according to a variant of the invention, the whole culture medium may be replaced by some fresh pre-warmed one after an incubation time of the virus, cationic poloxamer formulated or not with nanoparticles and target cells that can vary following the procedures commonly performed in cell biology (Harrisson M.A. et al in General Techniques of Cell Culture Ed: Cambridge University Press 1993, ISBN-10: 052157496X).

According to another variant of the invention, centrifugation step after the administration of the viral vector/ cationic poloxamer formulated or not with nanoparticles mixture can be performed. Practically an enhancement of the adjuvanted transduction efficiency may be observed by centrifuging the plate just after the administration of the vector (A.B. Bahnson et al.; J. Virol. Methods 1995, 54: 131-143).

According to the invention any kind of cell culture material or cell culture methods can be used respectively in 2 dimensions (2D) or onto 3 dimensional (3D) supports to transduce said 'target cells". 2D devices are defined as support that allow cells to be cultured in an adherent way (cells adhere at the bottom of the well), in suspension way (cells remains floating into the culture medium), or in co-culture (2 or more different kinds of cell types separated or not with an insert) such as treated or non-treated cell culture plates and dishes (from 1536-wells to 4-well cell culture plates, 35 mm, 60 mm, 90 mm, 100 mm petri dishes) or cell flasks (such as 25cm², 75cm² or 150cm²...). This invention may also be applied to 3D matrices such as hydrogel or solid 3D scaffolds of various compositions such as collagen, atelocollagen, glycosan, polystyrene, hyaluronic acid, polycaprolactone, polyethylene glycol.

According to yet another variant of the invention magnetic nanoparticles may be added to the mixture of target cells, cationic poloxamer and virus.

In that case where the cationic poloxamer is associated with magnetic nanoparticles, the method includes an optional additional step, wherein a permanent or oscillating or electro-mediated magnetic field can be applied to the experiment after the virus, the cells and the cationic poloxamers formulated or not with nanoparticles have been put in contact. This step can last from 10 seconds to 96 h, preferentially from 1 minute to 48 h. More preferentially it will last for 5 min to 4 h.

According to the invention said "target cells" can also be infected and/or transduced onto magnetic cell separation devices whether or not they use columns to support positive or negative cell selection. The term "cell selection" refers to immunomagnetic cell separation technologies used to isolate specific cell populations (such as Hematopoietic stem cells, T lymphocytes, B lymphocytes, Natural Killers, PBMC, splenocytes...) from variety of species (such as human, mouse, rat, non-human primate, rabbit, bovine...) with the help of a magnetic field. Accordingly, the invention may be used to infect or transduce cells either retained onto the separation device or right after the selection process; moreover, the cationic poloxamer formulated with magnetic nanoparticles can be used with viruses to retain the viral particles onto the magnetic separation devices and to further directly infect cells on the same device. This invention is of particular interest to infect various cell types from cell lines to primary cells such as Jurkat T cells, K562, cord blood primary CD34+ cells, bone marrow cells or human mesenchymal stem cells (Sanchez-Antequera Y., Blood. 2011 Apr 21;117(16):e171-81). When the cationic poloxamer formulated or not with magnetic nanoparticles is added onto the separation device, time of contact with the separation device covered by the scope of this invention ranges from 15 seconds to 1 week. More preferentially contact times should be comprised within 1min to 6 hours. More preferentially it should be comprised within 1min and 1h.

According to the invention said "target cell" can be any cell that can be targeted for transduction with a viral vector. The term "cell" as used in connection with the present invention can refer to a single and/or isolated cell or to a cell that is part of a multicellular entity such as a tissue, an organism or a cell culture. In other words, the method can be performed *in vivo, ex vivo* or *in vitro.* The cell can be a primary cell (the term "primary cell" as used herein is known in the art to refer to a cell that has been isolated from a tissue and has been established for growth *in vitro*) or established cell lines or a stem cell or a progenitor cell. The cell can be eukaryotic cell such as endothelial, epithelial, fibroblastic, hepatic, hematopoietic (lymphocytes, monocytes, macrophages natural killer dendritic cells etc.), muscle, nerve cells (all types of neurons such as cortical, hippocampal, sensory neurons, motor neurons, dorsal route ganglion, oligodendrocytes, cerebellar granule, neural stem cells and basket cells, Betz cells, medium spiny neurons, Purkinje cells, pyramidal cells, Renshaw cells, granule cells or anterior horn cells, glial cells astrocytes etc.), stem cells, embryonic stem cells, hematopoietic stem cells, germ cells, a tumor cells, a lymphoid cell lineage and somatic cells to name a few but not limited too. The cells can be prokaryotic (bacteria), plant cells, insect, yeast, or parasite cells. They can be differentiated or totipotent or omnipotent or multipotent or oligopotent or unipotent or pluripotent form. They can be embryonic stem cells or induced pluripotent stem cells (iPS) or differentiated from embryonic or iPS. The cells to be transduced can be non-permissive cells, hard-to-infect cells, or easy to infect cells. Tumor cells and cell lines as known in the art can be for example, but not limited to, lymphoma cell lines (such as, e.g, Jurkat, CEM, H9, Daudi, SUP-M2, KARPAS-299), dendritic cell lines (such as DC2.4, Mutu), natural killer cell lines (such as NK-92, KHYG-1), epithelial cell lines (such as NIH-3T3, COS, CHO, HEK293), pancreatic tumor cells (such as PANC-1), a stem cell line (KG1a), breast cancer cells (MCF-7, T74D, MDA-MB361), a neuroblastoma (such as SH-SY5Y, N2a), a fibrosarcoma (such as HT1080), an astrocytoma (such as 1321N1), endothelial cells (such as HUVEC, HMEC, HCAEC). An eukaryotic cell as used herein, can refer to any cell of a multi-cellular eukaryotic organism, including cells from animals like vertebrates.

Preferably, said target cell can be a mammalian cell. The term "mammalian cell" as used herein, is well known in the art and refers to any cell belonging to or derived from an animal that is grouped into the class of Mammalia.

In the method of the invention, the enhanced transduction of viruses with the cationic poloxamer can target a tissue or an organ as stated previously. The tissues can be for example, but not limited to, muscle, connective, epithelial or nervous tissues. Methods to obtain samples from various tissues and methods to establish primary cell lines and immortalized cell lines are well-known in the art (Freshney, R.I. *Culture of Animal Cells: A* *Manual of Basic Technique,* Fifth Edition, 2005 Ed: John Wiley & Sons, Inc.).

According to the invention, the cationic poloxamer will be based on a block copolymer containing a minimum of three blocks. By block it is meant a polymeric construct bearing either hydrophilic or hydrophobic properties. Preferentially, hydrophilic blocks refer to a poly(ethylene) oxide polymeric chain, whereas hydrophobic block preferentially refers to a poly(propylene) oxide polymeric backbone. The order and the succession of these different blocks defines the general structures of the polymers used as transduction adjuvant in this invention.

In the case where cationic poloxamer is based on the regular and linear poloxamer family, the first central block, identified as the hydrophobic/lipophilic block will be preferentially constituted by a poly(propylene) oxide polymeric chain. Said hydrophobic/lipophilic block will be surrounded by two more hydrophilic blocks, either similar or different, preferentially based on the poly(ethylene) oxide pattern. When the cationic poloxamer is based on the "reverse poloxamer (or Meroxapol)" family, the central block will be constituted by a poly(ethylene) oxide polymer surrounded by two hydrophobic blocks, identified as poly(propylene) oxide polymeric chains.

Such cationic poloxamer and reverse cationic poloxamer can be linear or X-shaped, these latest being known as poloxamines or reverse poloxamines according to the same definition. All those families of polymers will be described under the general name of cationic poloxamers in the rest of the document.

Due to their synthesis methodology which involves anionic polymerization, cationic poloxamers always present two hydroxyl (or alcohol) functions at the end of their backbone. Polymer chemists generally relate to it as "polymer endgroups".

In the present invention, these endgroups have been modified in order to introduce cationic moieties at both ends of the polymeric chain. A complete description of these modifications is detailed further in this document. It includes complete synthetic procedure characterization and biological evaluation.

In this document, the term cationic poloxamer refers thus to triblock copolymers composed by hydrophobic chains of polypropylene oxide, and hydrophilic chains of polyethylene oxide that are originally ended by hydroxyl functions and which have been subsequently modified using well-known organic chemistry procedures in order to introduce cationic organic functions at both ends of the polymeric chains.

The invention also relates to new cationic poloxamers that can be used in the method of the invention as well as the cationic poloxamers known from the prior art at the date of the invention such as cationic poloxamers described by G. Gyulain et al in eXPRESS Polym Letters, 2016, 10:216:26 or by Slobodkin et al. in US20100004313A1.

Thus, the invention also relates to novel linear or branched cationic triblock copolymers that can be represented by the following formula I or formula II:

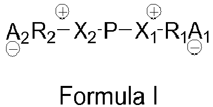

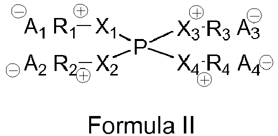

According to Formulas I and II, P can refer to the backbone of the triblock copolymer that can comprise hydrophobic chains of polypropylene oxide, and hydrophilic chains of polyethylene oxide.

According to Formula I, in the case of linear cationic poloxamer, P can be described according to the following Formula III a or b: 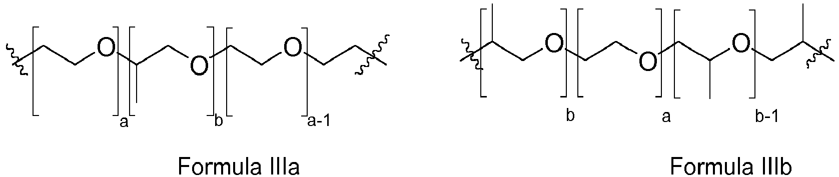 wherein Formula III covers the case of the regular cationic poloxamer (where the hydrophobic poly(propylene) oxide block is surrounded by two hydrophilic poly(ethylene) oxide blocks (Formula IIIa) and those of "reverse cationic poloxamer/meroxapol" (where the hydrophilic poly(ethylene) oxide block is surrounded by two hydrophobic poly(propylene) oxide blocks (Formula IIIb).

According to Formula II, that covers the case of modified X-shaped cationic poloxamer known as poloxamines, P can be described according to the following Formula IV a or b:

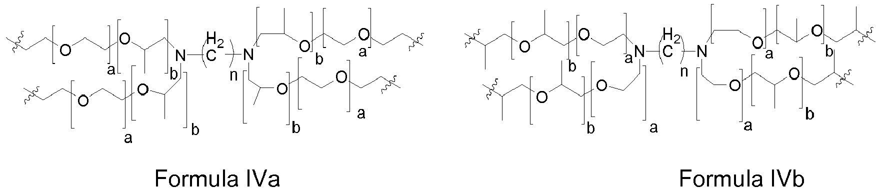

According to Formula I; II, IIIa, IIIb, Iva and IVb:
- *"a"* can represent the number of hydrophilic units repeated in the polymeric backbone, and is an integer that can range from 2 to 10000, preferentially, from 5 to 1000, more preferentially from 20 to 200;
- *"a-1"* can refer to the number *"a"* described before, in which 1 unit has been subtracted. Thus, it can range from 2 to 10000, preferentially from 5 to 1000, more preferentially from 20 to 200;
- *"b"* can represent the number of hydrophobic units - repeated all along the polymeric backbone and is an integer that can range from 2 to 1000, preferentially from 5 to 500, more preferentially from 15 to 80;
- ***"b-1"*** can refer to the number ***"b"*** described before, in which 1 unit has been subtracted. Thus, it can range from 2 to 1000, preferentially from 5 to 500, more preferentially from 15 to 80;
- **X₁, X₂, X₃** and **X₄** can refer to heteroatoms, preferentially chosen from Nitrogen, Phosphorous, Silicon and Sulphur. **X₁, X₂, X₃ and X₄** can be the same or different. **X₁**, **X₂, X₃ and X₄** are covalently bonded to respectively **R₁, R₂, R₃ and R₄;**
- **n** is an integer comprised between 1 to 20 more preferentially between 2 to 6;
- **R₁, R₂, R₃ and R₄** can be chosen so that entities **"-X₁-R₁", "-X₂-R₂", "-X₃-R₃"** and **"-X₄-R₄"** can be cationic. It is meant that **"-X₁-R₁", "-X₂-R₂", "-X₃-R₃"** and **"-X₄-R₄"** can contain one or several positive charge(s) in aqueous environment at pH ranging from 4 to 9, preferentially at physiological pH ranging from 6 to 8. That can enclose positive charges localized on heteroatoms, such as nitrogen, phosphorous, sulphur. **"-X₁-R₁", "-X₂-R₂", "-X₃**-**R₃"** and **"-X₄-R₄"** might share the same structure or be different according to the invention.

According to this **R₁, R₂, R₃ and R₄** can be, simultaneously or not:
- 1 to 6 hydrogen atoms, preferably 2 to 4.
- 1 to 8 heteroatoms, preferably 2 to 4, to be chosen between, nitrogen, phosphorous, sulphur and oxygen.
- 1 to 24, preferably 1 to 12, more preferentially 1 to 6, linear, branched and/or cyclic, saturated or unsaturated hydrocarbon group comprising from 1 to 24 carbon atoms, incorporating or not one or more heteroatoms such as oxygen, nitrogen, sulphur, phosphorous.
- 1 to 6, more preferentially 1 to 3 amino acid residues, natural or not.
- Any combination of these definitions

Consequently, and according to the invention **"-X₁-R₁", "-X₂-R₂", "-X₃-R₃"** and **"-X₄**-**R₄"** can be:
∘ Primary, secondary or tertiary amines cationic moiety. Amines derivates such as guanidines, hydrazines, guanidinium, hydrazinium can be preferred entities according to the invention;
∘ Organic quaternary phosphonium moieties such as for example substituted tri-n-butylphosphonium, substituted triphenylphosphonium, substituted triethylphosphonium;
∘ Quaternary ammonium salts based on a nitrogen atom covalently linked to 4 carbon moieties such as for example substituted quaternary trimethylammonium, substituted quaternary tri-n-butylammonium, substituted quaternary tri-n-octylammonium;
∘ Organic tertiary sulfonium salts based on a sulphur atom covalently linked to 3 carbon moieties such as for example substituted dimethyl sulfonium, substituted di-n-butylsulfonium, substituted di-*n*-octylsulfonium);
∘ Organic heterocycles bearing a net positive charge, delocalized or not on the cycle. By heterocycles it is meant an organic carbonated cyclic structure from 3 to 20, preferably from 3 to 8 carbon atoms, incorporating at least 1 to 6 similar or different heteroatoms such as oxygen, nitrogen, sulphur, phosphorous. These heterocycles may also include or not at least one unsaturation able to provide them an aromatic character, such as for example pyridine, and its cationic counterpart pyridinium; imidazole and its cationic counterpart imidazolium; triazole and its cationic counterpart triazolium; piperidine and its cationic counterpart piperidinium; morpholine and its cationic counterpart morpholinium;
∘ Basic amino-acids residue as a source of cationic charges, such as for example residues of lysine, arginine, histidine, ornithine, tryptophane, natural or not;
∘ Natural non-polymeric polyamines, such as for example spermine, spermidine or thermospermine derivatives that are not of polymeric nature.
∘ Furthermore, according to the invention any combination of the chemical structures described for **"-X₁-R₁", "-X₂-R₂", "-X₃-R₃"** and **"-X₄-R₄"** combined into a single cationic poloxamer ending groups are covered by the scope of the invention. Thus according to the invention **"-X₁-R₁", "-X₂-R₂", "-X₃-R₃"** and **"-X₄-R₄"** structures can include one or several carbon-based chains, linear or branched, incorporating or not heteroatoms, carbon rings, heterocycles.
   - **A₁, A₂, A₃ and A₄"** can represent the counter ions identical or different that can be chosen from one or several of the organic groups such as for example:
      ∘ Halogen-based anions, such as for example iodide, bromide, chloride or fluoride;
      ∘ Organic groups bearing a negative charge, centred or not on a carbon atom such as for example methanesulfonate, trifluoromethanesulfonate, trifluoroacetate, acetate, formate, para-toluenesulfonate, carbonate, hydrogenocarbonate;
      ∘ Inorganic anions, such as for example sulphate, phosphate, nitrate, hydrogenosulphate, hydrogenophosphate;
      ∘ Inorganic, non-coordinating anions, defined as anions that do not interact, or only slightly with their associated cations. We can cite examples such as tetrafluoroborate, hexafluorophosphate or perchlorate, carba-closo-dodecaborate;
      ∘ Boron-centered organic anions based on tetrakis[3,5-bis(trifluoromethyl)phenyl]borate backbone;
with the exception of F108 backbone, wherein a=132.7, b=50.3, a-1=131.7, and X1R1 =X2R2=NH2; and F127 backbone, wherein a=100.22, b=65.2, a-1 =99.22, and X1R1 =X2R2=NH2.

According to the invention **A₁, A₂, A₃ and A₄** can maintain neutrality of the whole polymer as incorporation of cationic moieties as ending groups of the cationic poloxamer chains implies the presence of anionic counterparts to preserve the neutrality of the whole molecular ensemble. As a consequence, the number of negative charges contained in **A₁, A₂, A₃ and A₄"** will be directly linked to the number of positive charges present on **"-X₁-R₁", "-X₂-R₂", "-X₃-R₃"** and **"-X₄-R₄"**. Furthermore, the nature of **"A"** can be dependent of the reactants used during the modifications of the cationic poloxamers. If it is not possible to use a reactant that combines the chemical properties needed for the polymer modification and the correct nature of **"A",** a salt metathesis reaction using commonly described experimental conditions (Gutowski K.E. et al J. Am. Chem. Soc., 2003, 125:6632-6633) will be set up.

According to the invention, the choice of **"*a*"** and **"*b*"** will modulate the structural composition of the described cationic poloxamer and thus, modify its physico-chemical properties. Among those properties can be highlighted the HLB (for hydrophilic lipophilic balance) a well-known semi-empirical value, which reflects the hydrophilic or lipophilic behaviour of commonly used surfactants. According to the invention, the Davies' definition of HLB (Davies JT, Proceedings of the International Congress of Surface Activity 1957, 426-438) extended to neutral poloxamer definition by Guo et al., (Guo X et al. Journal of Colloid and Interface Science 2006 298: 441-450) has been used.

According to the invention, it should be noted that both "regular" poloxamine (where polypropylene oxide blocks can be linked to the central amine functions) and "reverse poloxamine" (where the polyethylene oxide blocks are linked to the central amine functions) are also considered in this invention.

All the molecules described in Formula I, and II are also referred in this document as cationic poloxamer, without taking account of their linear or X-shaped or reversed character.

According to the invention, described cationic poloxamer can be constructed with values of **"*a*"** and **"*b*"** chosen so that the HLB value of the resulting polymer before cationic modifications can be from 1 to 100 more preferentially from 10 to 40, more preferentially from 15 to 30. This objective can be reached by a careful choice of the poloxamer starting material, commercially available from different suppliers, such as BASF or CRODA.

As it is well known that polymer synthesis, especially through anionic polymerization like in the present invention, cannot lead to molecules perfectly controlled, comprising an accurate number of repeated units, most of the time the obtainment of a "pure" form of such polymer at the end of the synthesis by the manufacturer can be conditioned by the quality of the purification methods enabled. In accordance it is really difficult, if not impossible by a person skilled in the art, to give exact values of the parameters related to molecular weight of the cationic poloxamers described. As outlined herein, synthesis of cationic poloxamers can result in a mixture of polymers with varying molecular weight. Thus, the term "average", in relation with molecular weight, or HLB, is a consequence of the technical inability to produce cationic poloxamers all having identical composition.

Thus, cationic poloxamers used can be presented as a mixture of cationic poloxamers, each showing variability as regards to their molecular weight and composition. Parameters such as molecular weight, HLB, and the values **"*a*"** and **"*b*"** in Formula IIIa, IIIb, IVa and IVb reflected this variability and have to be taken as characteristics of the mixture of cationic poloxamers used in this work.

According to the invention preferred structures of compounds of Formula I can be those disclosed in Figure I using generic linear cationic poloxamer structures: 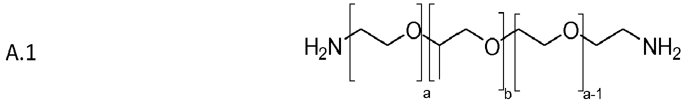 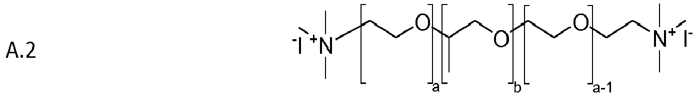 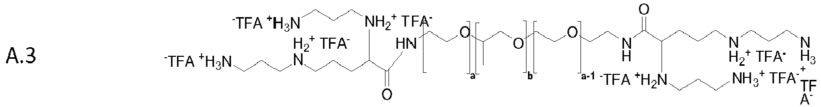 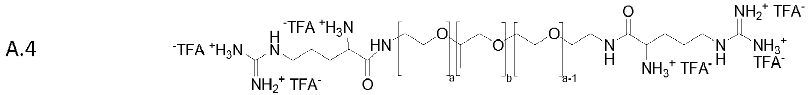 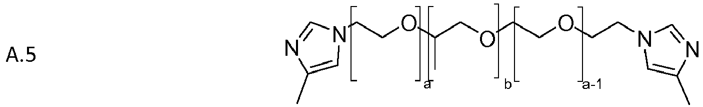 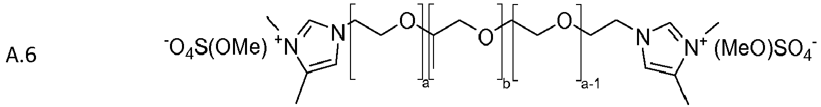 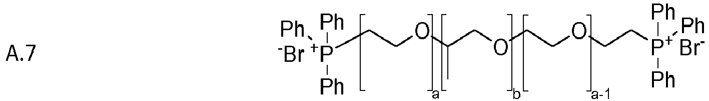 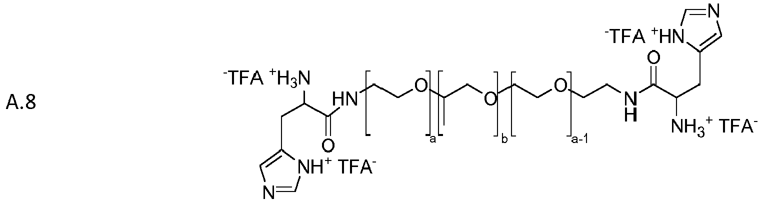 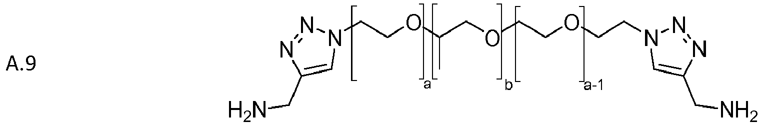 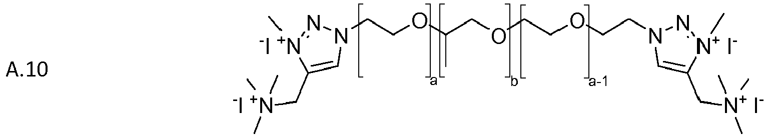 wherein values of **"a", "b"** and **"a-1" and "b-1"** can be as defined earlier in this text.

As previously said the following two molecules are excluded from the invention defined by formulas I, II, but are included in the invention relating to the method of transduction as cationic poloxamers:
- F108 backbone, wherein a=132.7, b=50.3, a-1=131.7, and X1R1 =X2R2=NH2; and
- F127 backbone, wherein a=100.22, b=65.2, a-1=99.22, and X1R1=X2R2=NH2.

According to the invention, cationic poloxamers described herein as well as any other cationic poloxamers, can be used as chemical adjuvant in transduction experiment. They can be associated with a viral vector before being put into contact with target cells. The concepts of contact, viral vector and target cells is in the present document.

Cationic poloxamers have the propensity to form gel at high concentrations. The cationic poloxamers described in the present invention can be supplied as a liquid or gel.

The cationic poloxamers can be dissolved at the given range of concentrations either in water, or in a suitable aqueous buffer routinely used in cell biology. Examples of such buffers can be: HEPES, DPBS (with or without calcium and/or magnesium), Tris, Carbonate, Acetate, Citrate. Choice of the dissolution concentration and of the dissolution medium can be made to maintain efficient and reproducible transduction efficiency, and to preserve the formulation relative to this invention in a liquid physical state. The cationic poloxamers can also be associated to different components such as surfactants able to form emulsions, gels, nanoemulsions or micelles.

The novel cationic poloxamers as well as the cationic poloxamers known from the prior art at the date of the invention can be associated or formulated with different kind of chemical molecules such as adjuvants capable of specifically targeting or binding to a determinant at the surface and/or inside the cells, optionally covalently or non-covalently attached to the compound corresponding to formula I, II, or to any other molecules contained in the composition comprising the compound of formula I, II, for example ligands of receptors expressed at the surface of the target cells, such as for example sugar, folate, transferrin, insulin, hormone, prostaglandins, peptide, antibody, metabolite, vitamins or any other molecule which can recognize an extracellular receptor, or an element of intracellular vectorization for targeting specific compartments such as the mitochondria, nucleus or cytoplasm, such as for example a nuclear or cytoplasm or mitochondrial localization signal such as for example a sugar, a peptide, a protein, an antibody, an antibody fragment, a ligand or a ligand fragment. Signalling molecules and molecules that may be involved in the signalling pathways inside of the target cells, such as activators or inhibitors of the protein kinase C or phospholipase signalling pathway, such as activators or inhibitors of the cellular metabolism pathways (AMPK signalling, insulin receptor signalling, glutamine metabolism pathway etc.), such as activators or inhibitors of the TLR-pathway (Toll-Like Receptor), the PRRs-pathway (Pattern Recognition Receptors), PAMPs-pathway, (Pathogen-Associated Molecular Patterns), DAMPs-pathway (Damage-Associated Molecular Patterns), AhR ligands (aryl hydrocarbon receptor ligands), the NOD-Like receptors (NLRs), the RIG-I-Like receptors (RLRs), cytosolic DNA sensors (CDS), the C-type lectin receptors (CLRs), activators or inhibitors of inflammasomes or inflammation or autophagy pathway, activators or inhibitors of the JAK/STAT pathway, activators or inhibitors of the ubiquitin and ubiquitin-like/proteasome pathway, activators or inhibitors of the PI3K/AKT pathway, activators or inhibitors of the tyrosine kinase, activators or inhibitors of the MAP kinase pathways, activators or inhibitors of the GPCR, calcium, cAMP signalling pathway, activators or inhibitors of the cell cycle, checkpoint control and DNA repair mechanisms, activators or inhibitors of the apoptosis pathway, regulators of the reactive oxygen species (ROS, NOS...), activators or inhibitors of immune checkpoints, regulators of protein synthesis and RNA degradation process, regulators of keys elements having a role at the chromatin or DNA level (i.e. activators or inhibitors of protein acetylation, histone lysine methylation, DNA methylation, nuclear receptor signalling...), regulators of microtubule and actin dynamics pathways, can also be considered in the present invention.

The cationic poloxamers can also be associated with molecules known to influence a transduction procedure. This covers a wide range of molecules such as polypeptides recombinant or not, liposomes, cationic lipids, cationic polymers such as polyethylenimine, protamine sulphate, polybrene, poly-L-lysine, DEAE Dextran, polylactic-co-glycolic acid, chitosans, neutral polymers such as non-modified poloxamer, polyethylene glycol and derivatives, anionic polymers such as starch and hyaluronic acid. These molecules can be added to the cells from 1 week before to 4 weeks after addition of the invention, more preferentially from 1 day before to 7 days after.

The main objective of this invention is to design new efficient adjuvants for transduction procedure. Magnetic nanoparticles have been used to enhance, synchronize and promote transduction. Accordingly, the cationic poloxamers were studied in combination with magnetic nanoparticles and it has been demonstrated in the present invention that the cationic poloxamers described in the present text can also act as stabilizing and synergistic agent for magnetic nanoparticles.

Indeed, the use of unmodified poloxamer at the surface of magnetic colloidal suspensions of beads is well-known. Due to their extraordinary biological compatibility, mainly due to the presence of the ethylene oxide chains, several examples associate it with iron-based magnetic based to enhance thus the propensity of these colloidal suspension to be used in biological environment, especially in *in vivo* procedures. However, in all cases, unmodified poloxamer needed to be associated with another type of molecules, polymeric or not, to provide the nanoparticle a zeta potential, or surface potential. It is indeed of prime importance that electrostatic repulsive interactions take place in colloidal suspension, so that the magnetic core may not aggregate leading to micrometric materials, which may have terrible consequences in the safety of hosts organisms. By extension, it is unlikely, that a magnetic nanoparticle coated with only a non-modified poloxamer remained stable in water over a long period of time, due to the lack of stabilizing charges. The positive charges added on the molecules of the invention as well as any other cationic poloxamers introduce the missing factor needed to stabilize by electrostatic interactions, magnetic iron core. According to the invention particles made that way can remain at the nanometric size range for weeks, while their counterpart coated with non-modified poloxamer fail to reach hours-long colloidal stability. This new interesting feature allowed the applicant to develop a new class of magnetic iron nanoparticles that shows an enhancement of the transduction efficiency when associated with a viral vector.

The invention also relates to a composition, preferably therapeutic or cosmetic or for life sciences, comprising at least one compound of formula I or II as previously defined.

In another aspect the invention relates to a composition comprising at least one compound of formula I or II and a virus.

In still another aspect the invention relates to a composition comprising at least one compound of formula I or II, and eventually a virus to transduce a target cell.

The Invention also relates to a kit comprising a cationic poloxamer alone or formulated as previously described and eventually further comprising a virus, and optionally, instructions of use.

The invention relates to transduced cells obtained by the method of claim 1 to 11. Said cells can be used in cell therapy.

Other features and advantages of the invention will be found in the following illustrative and non-exhaustive examples and in the accompanying Figures which illustrate the results of the tests carried out with the invention's compounds.
Figure I discloses preferred structures of compounds of Formula I using generic linear cationic poloxamer structures. Values of **"a", "b"** and **"a-1"** are as defined in this description.
Figure II discloses the detailed procedure for obtaining compound **Ib,** which is an intermediate of polymer I. Conditions i): TsCI, DMAP, DCM/pyridine RT; Conditions ii): NH3(MeOH), 80°C.
Figure III discloses the synthesis of compound 1e, which is an intermediate of polymer I; conditions i) NaOH, MeOH; Conditions ii): H₂, Raney Nickel EtOH; Conditions iii): Boc₂O, NaOH, THF/H₂O.
Figure IV discloses the synthesis of polymer I from 1b and 1e. Conditions i): DIC, HOBt, Et₃N, DCM/DMF; Conditions ii): TFA, DCM.
Figure V discloses the detailed procedure for the achievement of compound **II.** Conditions i):TsCI, DMAP, DCM/pyridine RT; Conditions ii): NH3(MeOH), 80°C. Conditions iii): Mel, DIPEA, DMF RT.
Figure VI discloses the detailed procedure for the achievement of compound III. Conditions i): CBr₄, PPh₃, DCM, RT; Conditions ii): PPh₃, DMF; 120°C.
Figure VII discloses the detailed procedure for the achievement of compound IV. Conditions i): NaN₃, DMF, 90°C; Conditions ii): Propargylamine, CuSO₄, Ascorbic acid, PPh₃, H₂O/DMSO RT; Conditions iii): Mel, DIPEA, DMF RT.
Figure VIII discloses the detailed procedure for the achievement of compound V. Conditions i): TsCI, DMAP, DCM/pyridine RT; Conditions ii): NH3(MeOH), 80°C. Conditions iii): Acetaldehyde, TosMIC, K₂CO3 DMF, RT. Conditions iv): Dimethyl sulfate, Toluene, RT. Figures IX discloses the detailed procedure for the achievement of compound **VI.** Conditions i): Boc₃R, DIC, HOBt, Et₃N, DCM/DMF RT; Conditions ii): Trifluoroacetic acid, DCM, RT.
Figure X discloses the detailed procedure for the achievement of compound **VII.** Conditions i): Boc₂Hist, DIC, HOBt, Et3N, DCM/DMF RT; Conditions ii): Trifluoroacetic acid, DCM, RT.
Figure XI discloses the results of the evaluation of cationic poloxamer in a transduction procedure using a lentiviral vector: NIH-3T3 cell line was infected with Lentivirus (MOI 1) in presence or not of various doses of cationic poloxamer. After 72 h incubation, % of GFP positive cells (A) and mean intensity (B) of transduced cells were evaluated by flow cytometry.
Figure XII discloses a second round of the evaluation of cationic poloxamer in a transduction procedure using a lentiviral vector: NIH-3T3 cell line was infected with Lentivirus (MOI 1) in presence or not of various doses of cationic poloxamers. After 72 h incubation, % of GFP positive cells (A) and mean intensity (B) of genetically modified cells were evaluated by flow cytometry.
Figure XIII discloses results of assays wherein BV2 and HEK-293 cell lines were transduced with Lentivirus encoding for GFP at the indicated MOI in presence or not of F108+polybrene, compounds 2b & II. After 72 h incubation, % of GFP positive cells (A) and mean intensity (B) of genetically modified cells were evaluated by flow cytometry.
Figure XIV discloses results of assays wherein Jurkat T cells were infected with Lentivirus (MOI 0.75) in presence or not of native or modified cationic poloxamers. After 72 h incubation, % of GFP positive cells (A) and mean intensity (B) of transduced cells were evaluated by flow cytometry.
Figure XV discloses results of assays wherein KG1a cell line was infected with Lentivirus (MOI 2) in presence or not of ranging doses of cationic poloxamers. After 72 h incubation, % of GFP positive cells (A) and mean intensity (B) of transduced cells were evaluated by flow cytometry.
Figure XVI A &B discloses results of assays wherein BV2, HeLa, Jurkat, C6, CLU-500 and KG1a cell lines were transduced with Lentivirus encoding for GFP at the indicated MOI in presence or not of cationic poloxamer 2b. After 72 h incubation, % of GFP positive cells (A) and mean intensity (B) of genetically modified cells were evaluated by flow cytometry.
Figure XVII A &B discloses results of assays wherein primary human CD34+ stem cells were transduced with Lentivirus encoding for GFP at the MOI of 5 in presence or not of ranging doses of modified cationic poloxamers. After 72 h incubation, % of GFP positive cells (A) and mean intensity (B) of genetically modified cells were evaluated by flow cytometry.
Figure XVIII A &B discloses results of assays wherein C6 and HEK-293 cell lines were transduced with Adenovirus encoding for GFP (AdGFP) with a MOI of 5 in presence or not of F108 and 2b. After 72 h incubation, % of GFP positive cells (A) and mean intensity (B) of genetically modified cells were evaluated by flow cytometry.
Figure XIX A &B discloses results of assays wherein HEK-293 cell line was transduced with AAV encoding for GFP (AAV-GFP) with 100000 genome copies per cell in presence or not of II. After 72 h incubation, % of GFP positive cells (A) and mean intensity (B) of genetically modified cells were evaluated by flow cytometry.
Figure XX A &B discloses results of assays wherein KG1a suspension cell line was transduced with Lentivirus encoding for GFP with a MOI of 5 associated with ViroMag magnetic nanoparticles (VM) cationic poloxamer 2b or ViroMag coated with a 5% w/v solution of 2b (VM+2b). After 72 h incubation, % of G GFP positive cells (A) and mean intensity (B) of genetically modified cells were evaluated by flow cytometry.

### EXAMPLES.

### a) Materials

Most of the solvents and reagents have been obtained from VWR Prolabo (Briare, France), Sigma-Aldrich SA (St Quentin-Fallavier, France), TCI Europe N V (Zwijndrecht, Belgium), and Bachem Biochimie SARL (Voisin-le-Bretonneux, France). Poloxamers used as starting materials are from BASF Performance polymers Europe (Rudolstadt, Germany) and from CRODA Industrial specialties Europe (Gouda, The Netherlands). All reagents purchased with reagent grade have been used without any further purification procedure, unless otherwise stated. Amino-acids used in this work all derived from natural one (*i.e. L-isomer),* unless otherwise stated.

### b) Methods

Thin layer chromatographies (TLC) are carried out on aluminum plates covered with silica gel 60 Fasa (Merck). The compounds are developed under UV light (254 nm), with iodine, by immersion in a ninhydrin developer (0.2% in butanol) followed by a stage of heating at 150°C. In the case of the compounds possessing a primary amine function, or by immersion in a cerium/concentrated molybdate (HO/ HSO₄/(NH₄)Mo.O.4H₂O/Ce(SO).3HO developer: 90/10/15/1) followed by a stage of heating at 110° C. in the case of the Sulphur-containing compounds.

The synthesized products are purified on chromatography columns on silica. The flash chromatography separations are carried out on silica gel 60 (230-400 mesh ASTM) (Merck).

Unless otherwise stated, polymers purifications have been processed through dialysis using membranes of different cutoffs sold by Sigma Aldrich or Spectrum Labs Europe BV (Breda, Netherlands). Dialyses have been processed against 4 liters of double distilled water. The medium was changed after 2 hours, 8 hours, 24 hours, 36 hours and 48 hours.

Freeze-drying has been carried out using an Heto PowerDry 3000 freeze dryer (ThermoFisher) on samples kept frozen 12h at -80°C.

### Size Exclusion Chromatography (SEC)

SEC Experiments have been processed following the Gel-permeation chromatography technique using an Agilent HPLC 1260 Chain (Agilent, Les Ulis France). This apparatus includes: an Agilent 1260 infinity Quaternary pump with manual injection valve, a refractive index detector RID Agilent 1260 Infinity, A UV detector with multiple wavelength Agilent infinity 1260, and a thermostated column's oven Agilent 1260 infinity. Data were collected and processed using Agilent Open Lab LC Chemstation software and its GPC extension. The stationary phase used in these experiments is an Agilent Biosec 3 column (100 Angstroms, 7.8^{∗}300 mm) thermostated at 40°C. Mobile phase is constituted by 0.5M acetate buffer, whose pH is corrected to 4.75. The mobile phase has been extensively degassed by sonication and filtered twice prior to use. Flow was adjusted at 1 mL/min to reach a working pressure ranging around 105 bars.

For analysis of the molecular mass properties of the polymer, a molecular weight calibration curve has been built using polyethylene oxide standards (Mw ranging from 20400 to 136 Da) provided by Agilent. Sample to be analyzed have been dissolved in double distilled water at a concentration of 1 mg/mL, and have been filtered before injection (syringe filter, 0.22 µm, Merck). The dilution water used for dissolving samples contained tetrahydrofuran (THF,10µL/100 mL of water) that was used as elution marker for all measurements.

Particle size and zeta potential measurements.

The mean hydrodynamic particle size and charge measurements for cationic poloxamers were performed using Dynamic Light Scattering (DLS) and Laser Doppler Velocimetry (LDV), respectively, using Malvern Nano ZS instrument and DTS software (Malvern Instruments, UK) in a water of Grade 2. Various amounts of cationic poloxamers were diluted in 100 µl of water and mixed with an equal volume of the same water containing DNA. The measurements were carried out in automatic mode and the results are presented as mean +/- SEM, n= 3. Each mean represents the average value of 30 measurements.

### Examples of synthesized molecules

A large ensemble of modified cationic poloxamers has been synthesized according to the method described in the present invention. All the molecules obtained in these examples derived from commercially available unmodified "hydrophilic" linear and regular poloxamer, which have a variety of value of HLB preferentially greater or close from 20 (see before for complete definition). The main structural features of the commercially available polymers used as starting material in this study are compiled in Table 1 below:

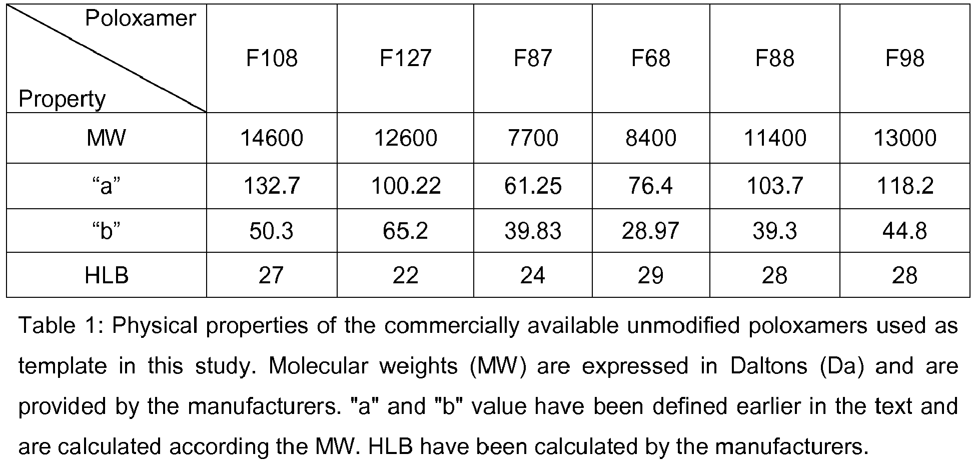

A large panel of cationic modifications is presented in the following examples, with different strategies applied to the different commercially available unmodified poloxamers described before. This led to different cationic polymers belonging to the present invention. Each of these polymers has been named after numbers (Tag), which are depicted in Table 2 hereafter:

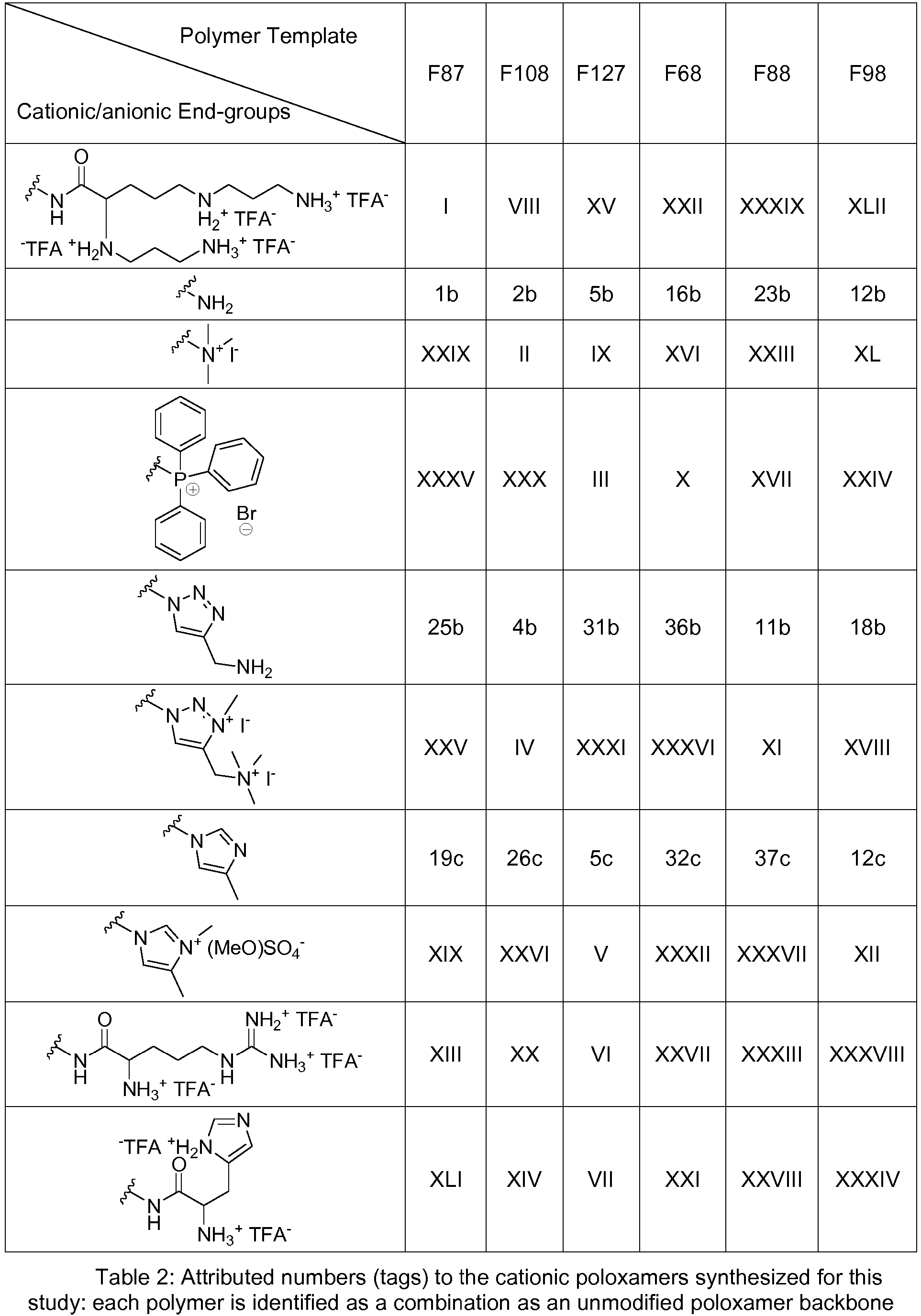

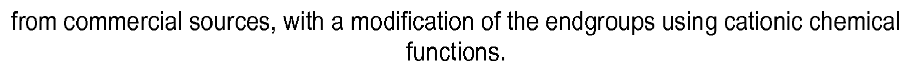

With the aim to keep the examples as simple as possible, only one example of each synthetic strategy will be described in its entirety. However, all these synthetic procedures have been applied with similar efficiencies for the obtaining of the compound described in Table 2.

Each synthetic step will thus be described completely for the chosen cationic poloxamer, while the results using the other polymeric backbones will be each time resumed in a table following the main procedure.

### Example 1: Synthesis of Bis-spermine-cationic poloxamer derivative

Example 1 deals with the introduction of spermine derivatives as endgroups on different hydrophilic backbones of the unmodified poloxamers. The chemical strategy will lead to compounds bearing the following general structure:

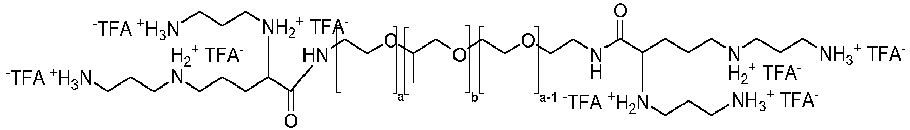

The physico-chemical features of the related compounds are dependent of the polymeric backbone chosen. They are condensed in the following Table 3.

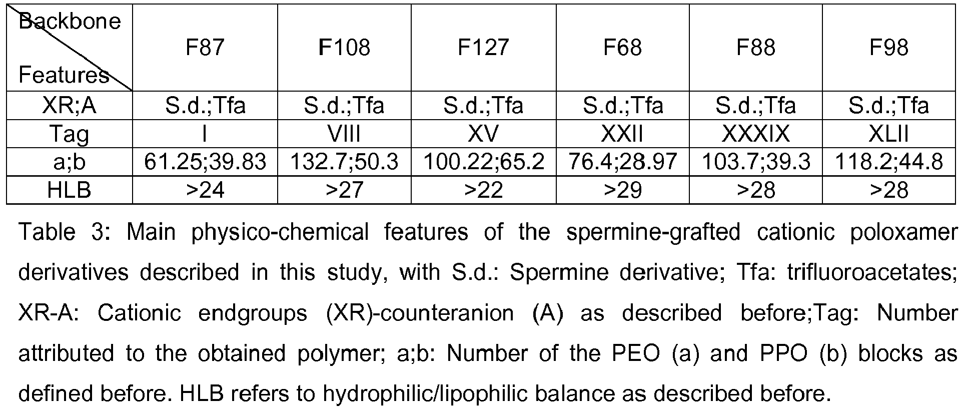

To illustrate the synthetic strategy, the detailed procedure for the obtaining of compound I is developed below. It started by the synthesis of compound 1b, described in Figure II:

### Step 1: Synthesis of bis-tosylated F87 1a.

Prior to the synthesis, 10g of F87 (Croda) are dissolved in 100 mL of MilliQ double distilled water and put in a 500 mL flask. The mixture is then frozen at -80°C overnight, before being freeze-dried.

Then, 3.36g (0.436 mmoles) are dissolved under argon in 86 mL of a mixture of dry dichloromethane with pyridine (volume ratio 73/13). The mixture is stirred vigorously before adding a catalytic amount of DMAP (TCI), and para-toluenesulfonic acid chloride (Sigma-Aldrich, 4.36 mmoles, 891 mg) portion wise over 45 minutes. The reaction is subsequently stirred for 48h at room temperature. The whole mixture is then concentrated under vacuum and the resulting yellowish gum is re-dissolved in DCM (50 mL). The organic extract is then washed twice with an aqueous saturated solution of NH₄Cl (2X100mL) and put aside. The combined washings are then extracted extensively with dichloromethane (5^{∗}50 mL), before combining the organic extracts and washing them twice with saturated NH₄Cl, twice with MilliQ water, and once with brine (each 150 mL). The obtained organic phase is then dried on magnesium sulphate, filtered and concentrated under vacuum to provide the title compound with has been used without further purification (3.17g).

Following Table 4 presents number and quantities of the starting material and name and quantity of the bis-tosylated product, in which m represents the mass of the compound while n the number of moles.

**Table 4**

| Backbone Material | F87 | F108 | F127 | F68 | F88 | F98 |
|---|---|---|---|---|---|---|
| Starting material (m; n) | **F87** (3.36g; mmol) | **F108** (16.8g; 1.15 mmol) | **F127** (11.7g; 0.929mmol) | **F68** (5.2g; 6.19mmol) | **F88** (2.2g; 0.193mmol) | **F98** (3.8g; 0.292mmol) |
| Isolated Product (m; n) | **1a** (3.17g ; 0.397mmol) | **2a** (15.8g; 1.06mmol) | **5a** (11.3g; 0.870mmol) | **16a** (4.45g; 0.512mmol) | **23a** (2.1g; 0.180mmol) | **12a** (2.7g; 0.203mmol) |

### Step 2: Synthesis of bis-amino F87 1b.

Compound **1b** (1.66g, 0.208 mmoles) is dissolved in 30 mL of a solution of methanol containing 2M of ammonia (TCI) under vigorous stirring. The solution is degassed with argon and then heated to reflux for 24h. Ammonia in methanol is then added (15 mL) and the mixture is refluxed for another 6h before being concentrated under vacuum. The resulting syrup is then dissolved in 20 mL of double distilled water, kept at 4°C for one night and introduced in a dialysis bag of 3.5 kDa cutoff (Spectrum Labs Europe). The dialysis is performed against 4 liters of double-distilled water with 5 medium-changes (2 hours, 8 hours, 24 hours, 36 hours and 48 hours). Final product is then recovered after lyophilisation (1.48g). This compound is of cationic nature. It thus belongs to the present invention.

Following Table 5 shows Tosyl displacement by ammonia step assessment: this table presents number and quantities of the starting material and name and quantity of the bis-amino product in which m represents the mass of the compound while n the number of moles.

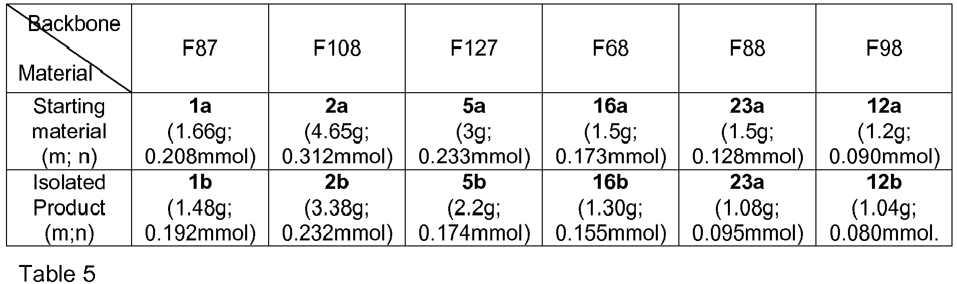

The achievement of the title compounds requires then the synthesis of ornithin-derivative 1e.
Synthesis of compound 1e, intermediate of I is summarized in Figure III:

### Step 3: Synthesis of 2,5-bis(2-cyanoethylamino)pentanoic acid 1c

Sodium hydroxide (1.78g, 44.5 mmoles, VWR) is finely grounded in a mortar and suspended in 150 mL of methanol in a round-bottom flask. L-Ornithine monohydrochloride (5g, 29.7 mmoles, TCI) is then introduced portion wise and stirred intensively for 1h30. The suspension is then filtered on a short pad of Celite (Sigma-Aldrich). The remaining clear filtrate is reintroduced in a flask and stirred, before 4.29 mL of acrylonitrile are added dropwise over 1h. After complete addition, the mixture is stirred at room temperature for 36h in the dark full until completion shown by TLC. When over, 12 mL of 37% HCI (VWR) are added carefully to the mixture creating a turbid precipitate upon stirring. The solid is collected by filtration and dried under vacuum (S1, 2.51 g). A few drops of 30% concentrated sodium hydroxide in water (VWR) are then added, resulting in a second solid formation, which is collected and dried the same way (S2, 1.13g). Analysis by TLC proved that S1 and S2 are the pure target product while remaining filtrate does not contain any targeted material. Global yield is 46% (3.64g, 13.7 mmoles).

### Step4: 5-amino-2-(3-aminopropylamino)pentanoic acid 1d

2,5-bis(2-cyanoethylamino)pentanoic acid 1c (2.51g, 9.44 mmoles) is suspended in 40 mL of absolute ethanol. The pH of the mixture is slightly increased with sodium hydroxide to help solubilization, before being transferred in a 50 mL steel lab autoclave (BüchiGlass; Uster Switzerland). A catalytic amount of Raney Nickel (Sigma Aldrich) is introduced and the autoclave is closed and put under 8 bars pressure of dihydrogen. The mixture is stirred that way for 12h. The gas is then evacuated and the mixture filtered on a short pad of Celite. After removal of the ethanol the resulting oil is used without further purification in the next step.

### Step 5: Synthesis of 2,5-bis[tert-butoxycarbonyl-[3-(tert-butoxycarbonylamino)propyljaminojpentanoic acid 1e

The crude product of step 4 is put in a three-necks round bottom flask containing 15 mL of a solution composed of tetrahydrofuran and water (Volume ratio of 5/1). The mixture is then cooled to 0°C with an ice/water bath. Under stirring, 4M NaOH in water (9.12 mL) and Boc₂O (11.4g, 57 mmoles) dissolved in THF (12 mL), are added in 4 times each alternatively. The whole addition process takes 30 minutes to be completed, and then, the reaction is let stirred 12 hours at room temperature. The reaction is then concentrated under vacuum, re-dispersed in 100 mL of 6M HCI, and extracted with diethyl ether (6X35 mL). The organic extracts are then dried on magnesium sulphate, filtered and concentrated. The product is purified on silica gel, using DCM/MeOH as mobile phase (gradient from 2% to 12% MeOH in DCM). After concentration, the target compound is isolated with 48% yield (2 steps, 2.91g, 4.53 mmoles).

This compound has been synthesized in high quantities and used associated with each bis-amino poloxamer following the strategy depicted in Figure IV.

### Step 6: Coupling between 1b and 1e

Compound 1e (32 mg, 4.8.10⁻² mmoles) is dissolved in 2 mL of dry DMF. DIC (9 µL, 5.6.10⁻² mmoles) and HOBt (8 mg, 5.6.10⁻² mmoles) are added successively to the mixture and stirred for 2h30. Poloxamer **1b** (127 mg) is then added directly in the stirring flask, and the reaction is pursued for 24h at room temperature, before being concentrated under high vacuum. The resulting syrup is then dissolved in 20 mL of double distilled water, kept at 4°C for one night and introduced in a dialysis bag of 3.5 kDa cutoff (Spectrum Labs Europe). The dialysis is performed against 4 liters of double-distilled water with 5 medium-changes (2 hours, 8 hours, 24 hours, 36 hours and 48 hours). Final product is then recovered after lyophilisation (123 mg).

Following Table 6 shows the Coupling with compound 1e step assessment: this table presents number and quantities of the starting material and name and quantity of the bis-protected product in which m represents the mass of the compound while n the number of moles.

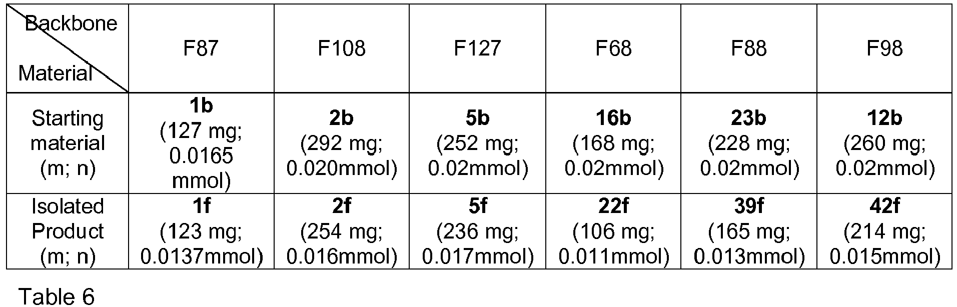

### Step 7: Synthesis of bis-substituted F87derivative I.

Compound **1f** (123 mg) is dissolved in 4 mL of a solution of dichloromethane. TFA is then introduced dropwise (VWR, 2 mL). The solution is degassed with argon and then stirred for 2h. Mixture is then concentrated under vacuum, and co-evaporated twice with DCM, three times with diethyl ether and dried one night under vacuum. The remaining residue is then resuspended in MilliQ water and introduced in a dialysis bag of 3.5 kDa cutoff (Spectrum Labs Europe). The dialysis is performed against 4 liters of double-distilled water with 5 medium-changes (2 hours, 8 hours, 24 hours, 36 hours and 48 hours). Final product is then recovered after lyophilisation (124 mg).
Following Table7 shows Boc-deprotection step assessment: this table presents number and quantities of the starting material and name and quantity of the bis-spermine product in which m represents the mass of the compound while n the number of moles

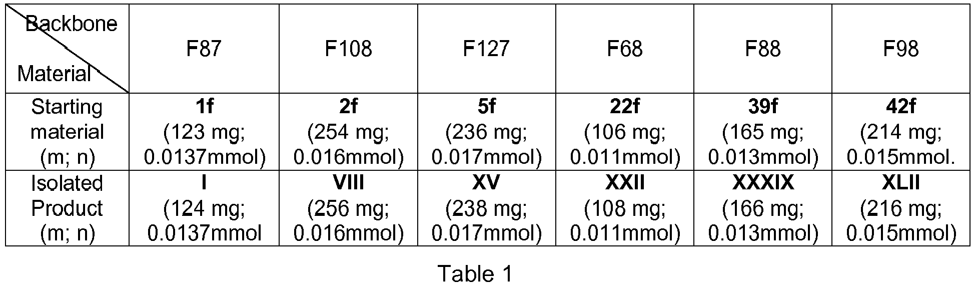

### Example 2: Synthesis of Bis-trimethylammonium-cationic poloxamer derivative

Example 2 deals with the introduction of trimethyl ammonium moieties as endgroups on different hydrophilic unmodified poloxamers. The chemical strategy will lead to compounds bearing the general structure depicted below:

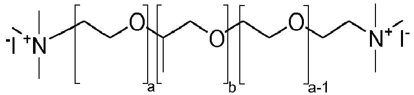

### Structure of trimethylammonium grafter cationic poloxamer derivatives

The physico-chemical features of the related compounds are dependent of the polymeric backbone chosen. They are condensed in the following Table 8.

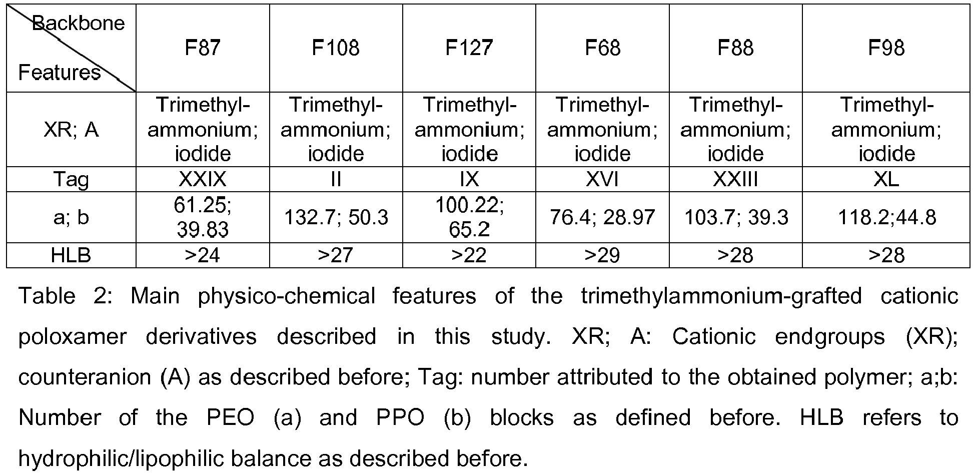

The synthetic strategy, the detailed procedure for the achievement of compound II is developed in Figure V.

### Step 1: Synthesis of bis-tosylated F108 2a.

Prior to the synthesis, 20g of F108 (Croda) are dissolved in 200 mL of MilliQ double distilled water and put in a 1000 mL flask. The mixture is then frozen at -80°C overnight, before being freeze-dried.

Compound **2a** is obtained following the strategy and proportions used in the synthesis of compound **1a,** starting from 16.8g of F108 to get 15.8g of **2a.** See Table 4 for detailed results.

### Step 2: Synthesis of bis-amino F108 2b.

Compound **2b** is obtained following the strategy and proportions used in the synthesis of compound **1b,** starting from 4.65g of **2a** to get 3.38g of **2b.** This compound is of cationic nature. It thus belongs to the present invention and will be evaluated further in the document.

See Table 5 for detailed results.

### Step 3: Synthesis of bis-trimethyl-ammonium F108 derivative II.

In a round-bottom flask under argon are introduced 300 mg (2.06.10⁻² mmoles) of compound **2b,** in 8 mL of dry DMF. After complete dissolution within a few minutes are added DIPEA (Sigma-Aldrich, 60 µL, 3.44.10⁻¹ mmoles) and methyl iodide (Sigma-Aldrich, 75 µL, 1.21 mmoles) and the resulting mixture is stirred for 48h at room temperature. After removal of the solvent, the remaining residue is then resuspended in MilliQ water and introduced in a dialysis bag of 3.5 kDa cutoff (Spectrum Labs Europe). The dialysis is performed against 4 liters of double-distilled water with 5 medium-changes (2 hours, 8 hours, 24 hours, 36 hours and 48 hours). Final product is then recovered after lyophilisation (290 mg).

Following the Table 9 shows Trimethylation step assessment: this table presents number and quantities of the starting material and name and quantity of the bis-trimethylated product in which m represents the mass of the compound while n the number of moles.

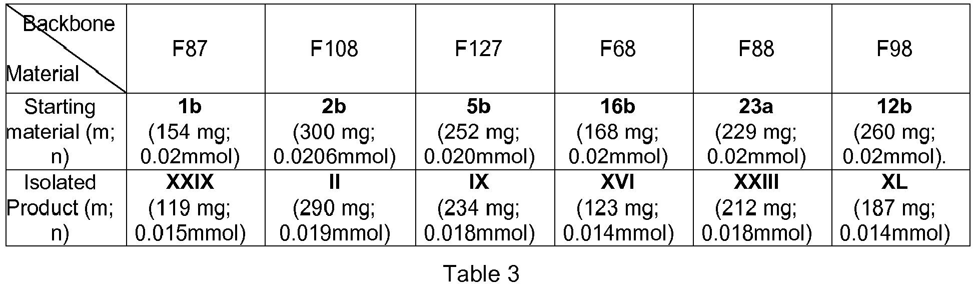

### Example 3: Synthesis of TriphenylPhosphonium-cationic poloxamer derivatives

Example 3 deals with the introduction of triphenylphosphonium moieties as endgroups on different hydrophilic unmodified poloxamers. The chemical strategy will lead to compounds bearing the general structure depicted below:

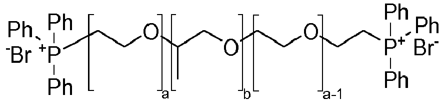

### General structure of triphenylphosphonium grafted cationic poloxamers derivatives

The physico-chemical features of the related compounds are dependent of the polymeric backbone chosen. They are condensed in the following Table 10.

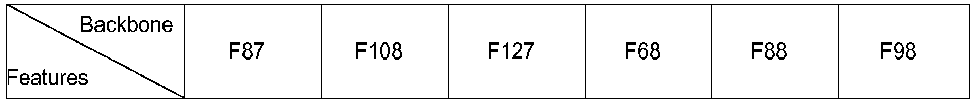

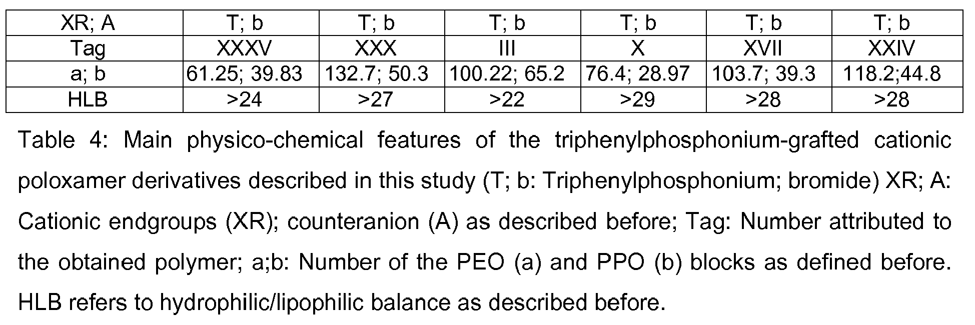

To illustrate the synthetic strategy, the detailed procedure for the achievement of compound **III** is developed in Figure VI.

### Step 1: Synthesis of bis-Brominated F127 3a.

Prior to the synthesis, 20g of F127 (Croda) are dissolved in 200 mL of MilliQ double distilled water and put in a 1000 mL flask. The mixture is then frozen at -80°C overnight, before being freeze-dried.

In a 100 mL round-bottom flask, are dissolved 5.45g of F127 (0.432 mmoles) under an argon atmosphere. Carbon tetrabromide (Sigma, 2.46g, 7.42 mmoles) and triphenyl phosphine (Sigma, 2.13g, 8.14 mmoles) are added simultaneously in three portions over a 1-hour period. The mixture is vigorously stirred during 48h at room temperature before removing dichloromethane under vacuum. The resulting solid is then resuspended in 50 mL of MilliQ water and kept at 4°C for 12 hours. After removal of the solid by centrifugation, the title product is isolated without further purification by lyophilization (1.62g).

Following Table 5 shows bromination step assessment: this table presents number and quantities of the starting material and name and quantity of the bis-brominated product in which m represents the mass of the compound while n the number of moles.

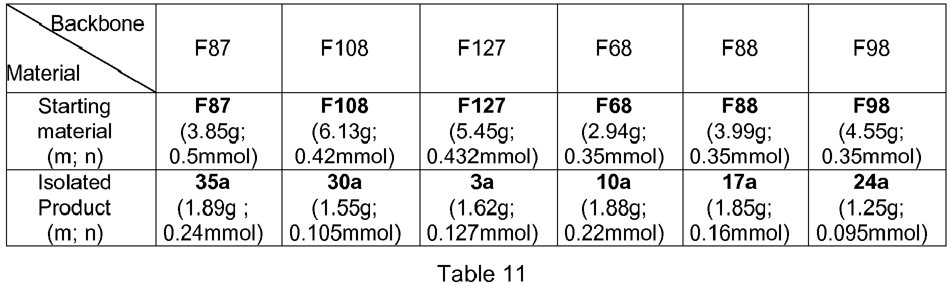

### Step 2: Synthesis of bis-phosphonium F127 derivative III.

1g (0.078 mmoles) of compound **3a** is dissolved in 30 mL of dry DMF. PPh₃ (1.6 mmoles 420 mg) is subsequently added under argon and the reaction is heated to 120°C in an oil bath while keeping an argon atmosphere. After 36h of stirring, the reaction is cooled down to RT, and concentrated under vacuum. The resulting syrup is washed extensively with several portions of hexane giving a solid which is then dried 12h under high vacuum. The remaining residue is then resuspended in MilliQ water and introduced in a dialysis bag of 3.5 kDa cutoff (Spectrum Labs Europe). The dialysis is performed against 4 liters of double-distilled water with 5 medium-changes (2 hours, 8 hours, 24 hours, 36 hours and 48 hours). Final product is then recovered after lyophilisation (654 mg).
Following Table 62 shows Phosphonium introduction step assessment: this table presents number and quantities of the starting material and name and quantity of the bis-phosphonium product in which m represents the mass of the compound while n the number of moles.

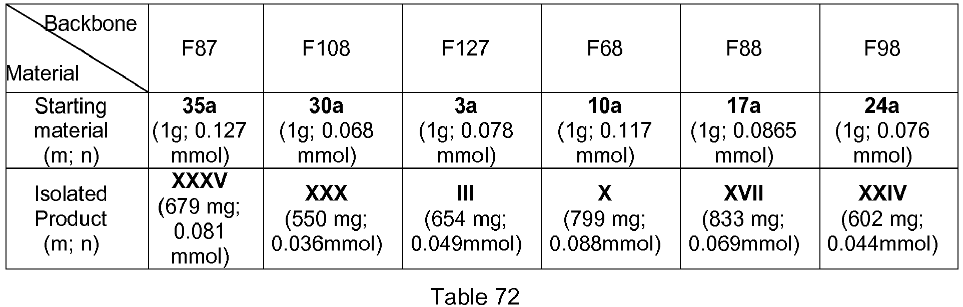

### Example 4: Synthesis of Bis-1-methyl-5-[(trimethyl-lambda5-azanyl)methyl]-1lambda5,2,3-triazacyclopenta-1,4-diene (MTAMTD) - cationic poloxamer derivatives

Example 4 deals with the introduction of 1-methyl-5-[(trimethyl-lambda5-azanyl)methyl]-1lambda5,2,3-triazacyclopenta-1,4-diene (MTAMTD) moieties as endgroups on different hydrophilic unmodified poloxamers. The chemical strategy will lead to compounds bearing the following general structure:

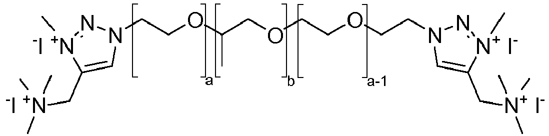

### General structure of MTAMTD-grafted cationic poloxamer derivatives

The physico-chemical features of the related compounds are dependent of the polymeric backbone chosen. They are condensed in the following Table 13.

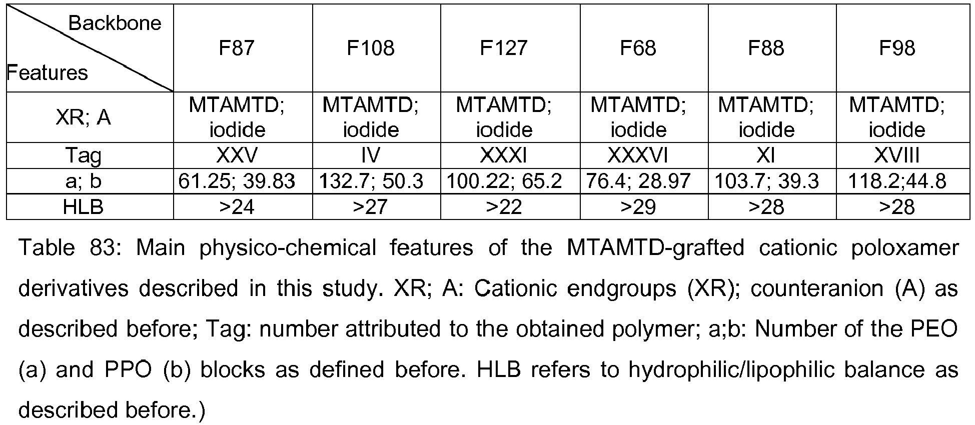

To illustrate the synthetic strategy, the detailed procedure for the achievement of compound **IV** is developed in Figure VII.

### Step 2: Synthesis of bis-azido F108 4a.

Compound **2a** (7.44 g, 0.5 mmoles) described earlier in this document, is dissolved under argon in 30 mL of dry DMF. Sodium azide (6 mmoles, 390 mg) is then introduced and the mixture is stirred at 90 °C for 3 days. After cooling down, DMF is removed using a rotary evaporator. The remaining residue is then resuspended in MilliQ water and introduced in a dialysis bag of 3.5 kDa cutoff (Spectrum Labs Europe). The dialysis is performed against 4 liters of double-distilled water with 5 medium-changes (2 hours, 8 hours, 24 hours, 36 hours and 48 hours). Final product is then recovered after lyophilisation (6.18 g).
Following Table 14 shows Azido introduction step assessment: this table presents number and quantities of the starting material and name and quantity of the bis-azido product in which m represents the mass of the compound while n the number of moles

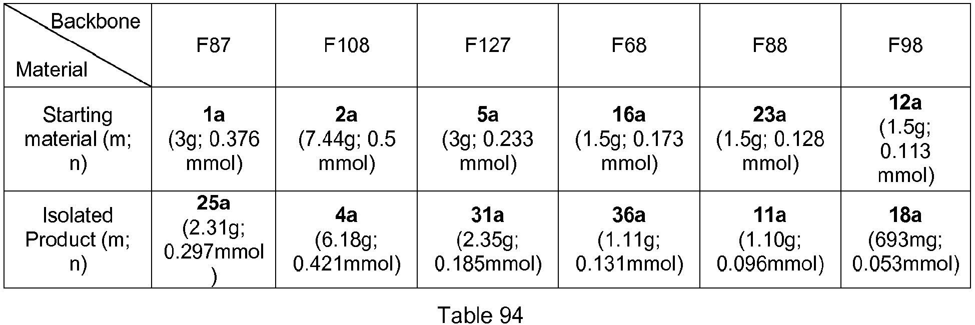

### Step 2: Synthesis of bis-1 H-triazol-4-ylmethanamine F108 4b.

Copper sulphate heptahydrate (VWR, 0.012 mmoles, 3 mg), ascorbic acid (Sigma, 0.06 mmoles, 12 mg) and triphenylphosphine (0.012 mmoles 3mg) are mixed together under stirring in 1 mL of dry DMSO. While this solution is stirring (15 minutes), compound **4a** (2g, 0.136 mmoles) is dissolved in 4 mL of a 3/1 mixture of water/DMSO. After complete dissolution of the polymer the copper-based yellowish solution is added to the reaction flask and stirred with the starting material for 12h at room temperature. The reaction is then diluted in 10 mL of water and extracted with 4X20 mL of DCM. The organic extracts are then combined, dried on magnesium sulphate, filtered and concentrated under vacuum. The remaining residue is then resuspended in MilliQ water and introduced in a dialysis bag of 3.5 kDa cutoff (Spectrum Labs Europe). The dialysis is performed against 4 liters of double-distilled water with 5 medium-changes (2 hours, 8 hours, 24 hours, 36 hours and 48 hours). Final product is then recovered after lyophilisation (1.72 g). This compound is of cationic nature. It thus belongs to the present invention.
Following Table 15 shows Huysgen-click reaction step assessment: this table presents number and quantities of the starting material and name and quantity of the products bearing triazole rings in which m represents the mass of the compound while n the number of moles.

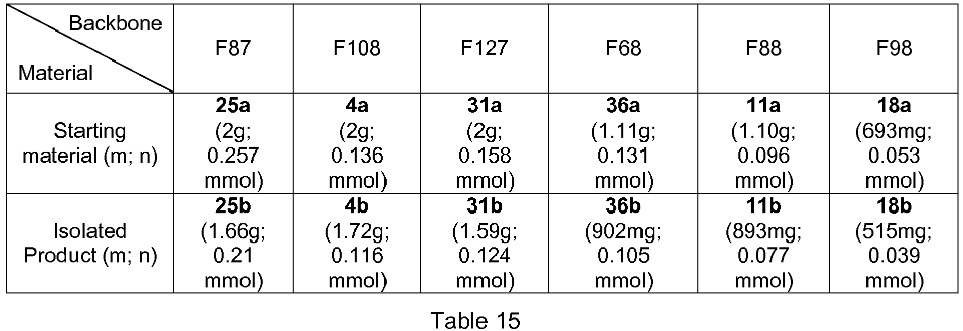

### Step 3: Synthesis of bis-1-methyl-5-[(trimethyl-lambda5-azanyl)methyl]-1lambda5,2,3-triazacyclopenta-1,4-diene F108 derivative IV.

In a round-bottom flask under argon are introduced 600 mg (4.05.10⁻² mmoles) of compound **4b,** in 18 mL of dry DMF. After complete dissolution within a few minutes are added DIPEA (Sigma-Aldrich, 120 µL, 6.88.10⁻¹ mmoles) and methyl iodide (Sigma-Aldrich, 150 µL, 2.42 mmoles) and the resulting mixture is stirred for 48h at room temperature. After removal of the solvent, the remaining residue is then resuspended in MilliQ water and introduced in a dialysis bag of 3.5 kDa cutoff (Spectrum Labs Europe). The dialysis is performed against 4 liters of double-distilled water with 5 medium-changes (2 hours, 8 hours, 24 hours, 36 hours and 48 hours). Final product is then recovered after lyophilisation (501 mg).

Following Table 16 shows methylation reaction step assessment: this table presents number and quantities of the starting material and name and quantity of the products bearing methylated triazolium rings in which m represents the mass of the compound while n the number of moles.

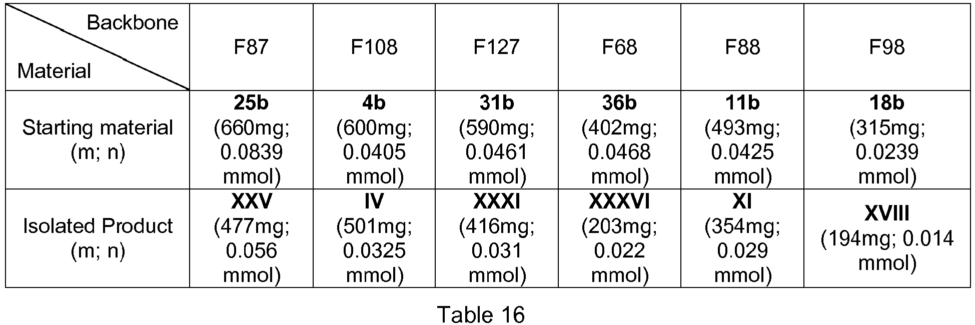

### Example 5: Synthesis of Bis-3,4-dimethyl-1H-imidazolium cationic poloxamer derivatives

Example 5 deals with the introduction of 3,4-dimethyl-1 H-imidazolium moieties as endgroups on different hydrophilic unmodified poloxamers. The chemical strategy will lead to compounds bearing the general structure depicted below:

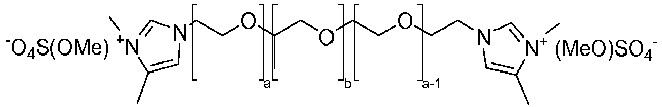

### General structure of 3,4-dimethyl-1 H-imidazolium -grafted cationic poloxamer derivatives

The physico-chemical features of the related compounds are dependent of the polymeric backbone chosen. They are condensed in the following Table 17.

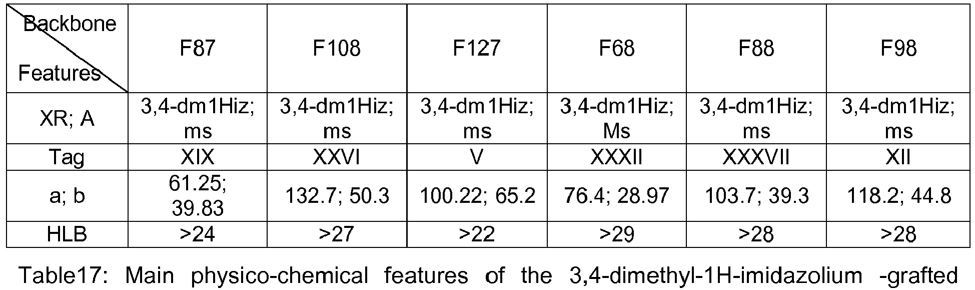

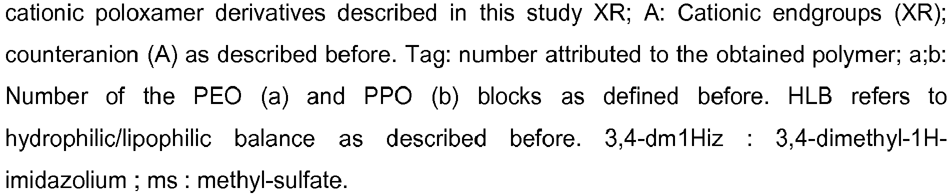

To illustrate the synthetic strategy, the detailed procedure for the achievement of compound **V** is developed in Figure VIII.

### Step 1: Synthesis of bis-tosylated F127 5a.

Prior to the synthesis, 20g of F127 (Croda) are dissolved in 200 mL of MilliQ double distilled water and put in a 1000 mL flask. The mixture is then frozen at -80°C overnight, before being freeze-dried.

Compound **5a** is obtained following the strategy and proportions used the synthesis of compound **1a,** starting from 11.7g of F127 to get 11.3g of **5a.**

See Table 4 for detailed results.

### Step 2: Synthesis of bis-amino F127 5b.

Compound **5b** is obtained following the strategy and proportions used the synthesis of compound **1b,** starting from 3 g of **5a** to get 2.2 g of **5b.** This compound is of cationic nature. It thus belongs to the present invention.

See Table 5 for detailed results.

### Step 3: Synthesis of bis-methylimidazole F127 5c.

Compound **5b** (1g, 7.93.10⁻² mmoles) is dissolved in 10 mL of dry DMF under an argon atmosphere. After complete dissolution, acetaldehyde (TCI, 1.6 mmoles, 100 µL) is introduced in the mixture, which is consequently stirred for 3 hours at room temperature. Potassium carbonate (VWR, 0.47 mmoles, 65 mg) and para-toluenesulfonic isocyanide (Sigma, 0.47 mmoles, 91 mg) are added subsequently and the reaction is kept under stirring for 24 hours at room temperature. At the end of the reaction the solvent is evaporated under vacuum, and then co-evaporated several times with ethyl acetate. The remaining residue is then resuspended in MilliQ water and introduced in a dialysis bag of 3.5 kDa cutoff (Spectrum Labs Europe). The dialysis is performed against 4 liters of double-distilled water with 5 medium-changes (2 hours, 8 hours, 24 hours, 36 hours and 48 hours). Final product is then recovered after lyophilisation (831 mg). This compound is of cationic nature. It thus belongs to the present invention.
Following Table 18 shows VanLeusen cyclisation step assessment: this table presents number and quantities of the starting material and name and quantity of the products bearing imidazoles rings in which m represents the mass of the compound while n the number of moles.

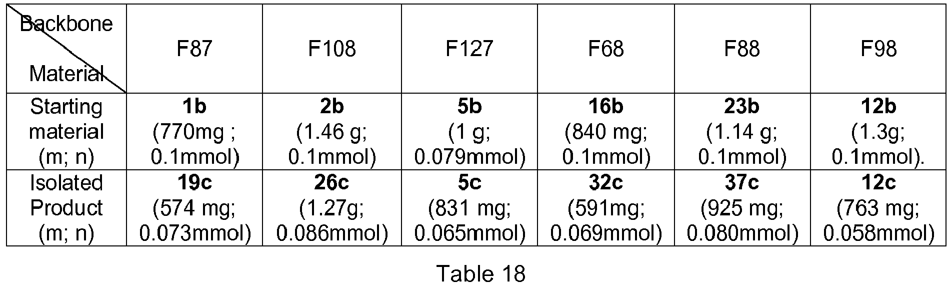

### Step 4: Synthesis of bis-methylimidazolium salt F127 V.

Compound **5c** (412 mg) is dissolved in 20 mL of dry toluene under argon. The flask is then cooled to 0°C using an ice-bath. While stirring intensively, dimethyl sulfate (Sigma, 6.3 mmoles, 600 µL) is introduced in the mixture carefully so that temperature inside the flask does not rise above 40 °C. After complete addition, the reaction is stirred at room temperature for two more days, concentrated under vacuum and then co-evaporated several times with diethyl ether and ethyl acetate. The remaining residue is then resuspended in MilliQ water and introduced in a dialysis bag of 3.5 kDa cutoff (Spectrum Labs Europe). The dialysis is performed against 4 liters of double-distilled water with 5 medium-changes (2 hours, 8 hours, 24 hours, 36 hours and 48 hours). Final product is then recovered after lyophilisation (274 mg).

Following Table 19 shows Imidazole methylation step assessment. this table presents number and quantities of starting materials and name and quantity of the products bearing methylated imidazolium rings in which m represents the mass of the compound while n the number of moles.

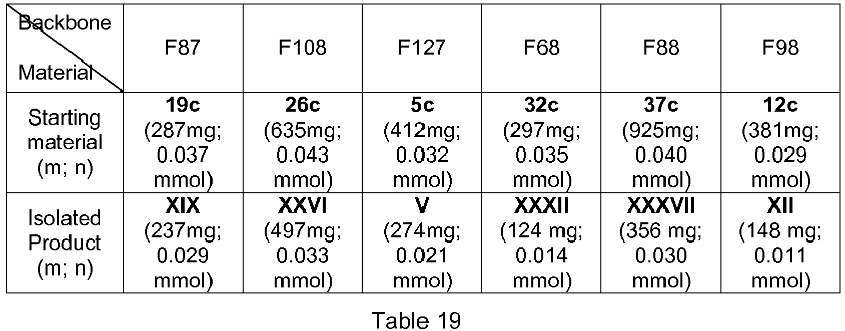

### Example 6: Synthesis of Bis-arginine-based cationic poloxamers derivatives

Example 6 deals with the introduction of arginine moieties as endgroups on different hydrophilic unmodified poloxamers. The chemical strategy will lead to compounds bearing the general structure depicted below:

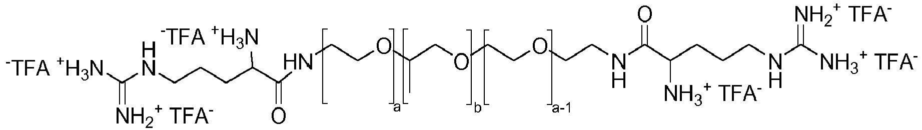

### General structure of cationic poloxamers derivatives bearing arginine moieties

The physico-chemical features of the related compounds are dependent of the polymeric backbone chosen. They are condensed in the following Table 20.

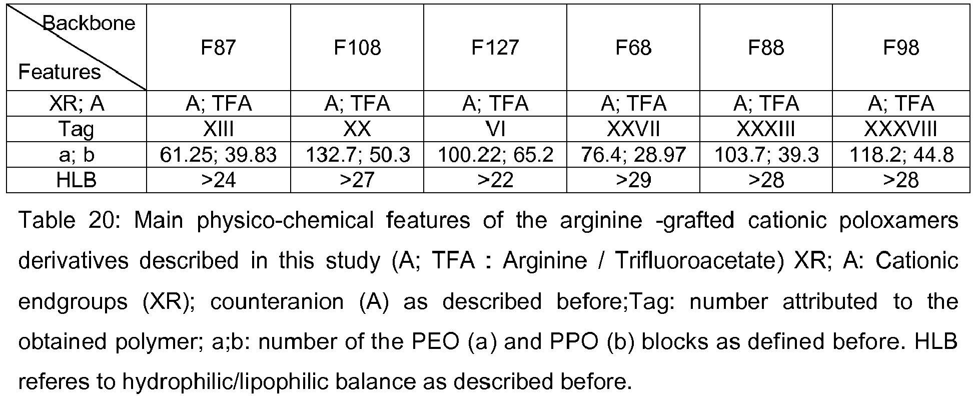

To illustrate the synthetic strategy, the detailed procedure for the achievement of compound **VI** is developed in Figure IX.

### Step 1: Coupling between 5b and Boc₃Arg

Boc₃Arg (Bachem, 30 mg, 6.4.10⁻² mmoles) is dissolved in 2 mL of dry DMF. DIC (15 µL, 9.6.10⁻² mmoles) and HOBt (17 mg, 13.10⁻¹ mmoles) are added successively to the mixture and stirred for 2h30. Cationic poloxamer **5b** (200 mg) is then added directly in the stirring flask, with triethylamine (TCI, 4.10⁻² mmoles, 6 µL) and the reaction is pursued for 24h at room temperature, before being concentrated under high vacuum. The resulting syrup is then dissolved in 20 mL of double distilled water, kept at 4°C for one night and introduced in a dialysis bag of 3.5 kDa cutoff (Spectrum Labs Europe). The dialysis is performed against 4 liters of double-distilled water with 5 medium-changes (2 hours, 8 hours, 24 hours, 36 hours and 48 hours). Final product **6a** is then recovered after lyophilisation (198 mg).
Following Table 21 shows Coupling with protected Arginine step assessment: this table presents number and quantities of starting materials and name and quantity of the products bearing protected arginine moieties in which m represents the mass of the compound while n the number of moles.

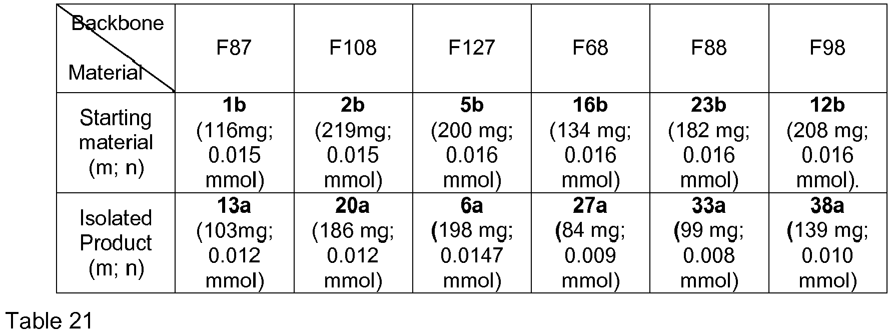

### Step2: Synthesis of bis-arginine substituted F127derivative VI.

Compound **6a** (198 mg) is dissolved in 4 mL of a solution of dichloromethane. TFA is then introduced dropwise (VWR, 1 mL). The solution is degassed with argon and then stirred for 2h. Mixture is then concentrated under vacuum, and co-evaporated twice with DCM, three times with diethyl ether and dried one night under vacuum. The remaining residue is then resuspended in MilliQ water and introduced in a dialysis bag of 3.5 kDa cutoff (Spectrum Labs Europe). The dialysis is performed against 4 liters of double-distilled water with 5 medium-changes (2 hours, 8 hours, 24 hours, 36 hours and 48 hours). Final product is then recovered after lyophilisation (194 mg).

Following Table 22 shows deprotection of protected Arginine step assessment: this table presents number and quantities of starting materials and name and quantity of the products bearing unprotected arginine moieties in which m represents the mass of the compound while n the number of moles.

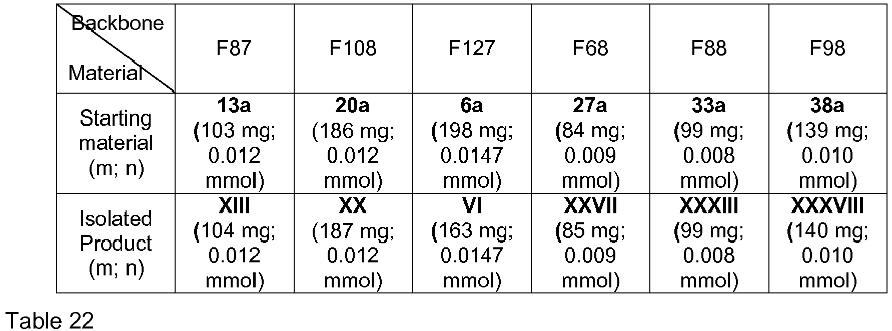

### Example 7: Synthesis of Bis-histidine-based cationic poloxamers derivatives

Example 7 deals with the introduction of histidine moieties as endgroups on different hydrophilic unmodified poloxamers. The chemical strategy will lead to compounds bearing the general structure depicted below:

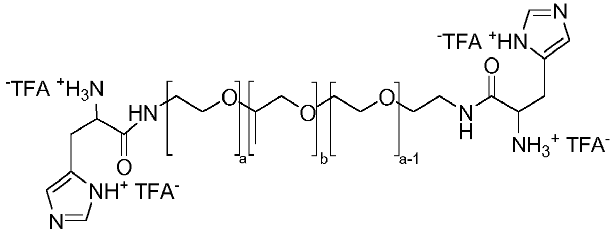

### General structure of histidine-based cationic poloxamers derivatives

The physico-chemical features of the related compounds are dependent of the polymeric backbone chosen. They are condensed in the following Table 23.

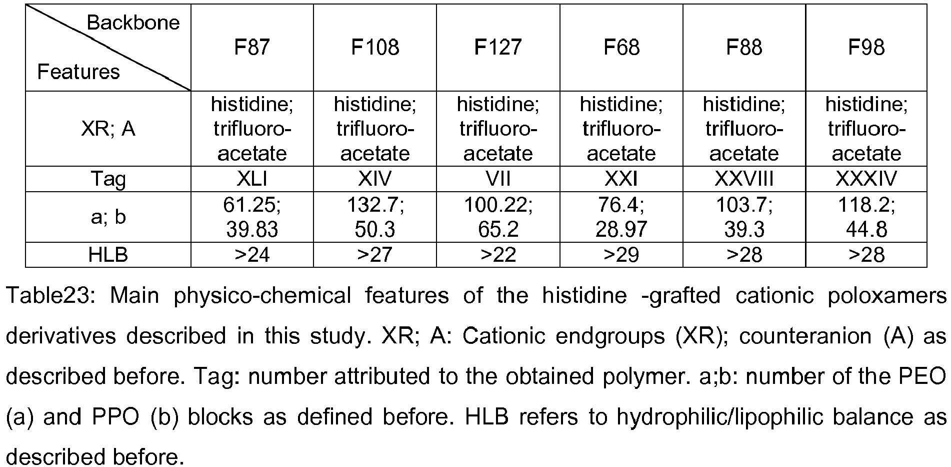

To illustrate the synthetic strategy, the detailed procedure for the achievement of compound **VII** is developed in Figure X.

### Step 1: Coupling between 5b and Boc₂Hist

Di-Boc-Histidine dicyclohexylammonium salt (Sigma, 34 mg, 6.4.10⁻² mmoles) is dissolved in 2 mL of dry DMF. DIC (15 µL, 9.6.10⁻² mmoles) and HOBt (17 mg, 13.10⁻¹ mmoles) are added successively to the mixture and stirred for 2h30. Cationic poloxamer **5b** (200 mg) is then added directly in the stirring flask, with triethylamine (TCI, 4.10⁻² mmoles, 6 µL) and the reaction is pursued for 24h at room temperature, before being concentrated under high vacuum. The resulting syrup is then dissolved in 20 mL of double distilled water, kept at 4°C for one night and introduced in a dialysis bag of 3.5 kDa cutoff (Spectrum Labs Europe). The dialysis is performed against 4 liters of double-distilled water with 5 medium-changes (2 hours, 8 hours, 24 hours, 36 hours and 48 hours). Final product 6a is then recovered after lyophilisation (198 mg).
Following Table 24 shows coupling with protected histidine step assessment: this table presents number and quantities of starting materials and name and quantity of the products bearing protected histidine moieties in which m represents the mass of the compound while n the number of moles.

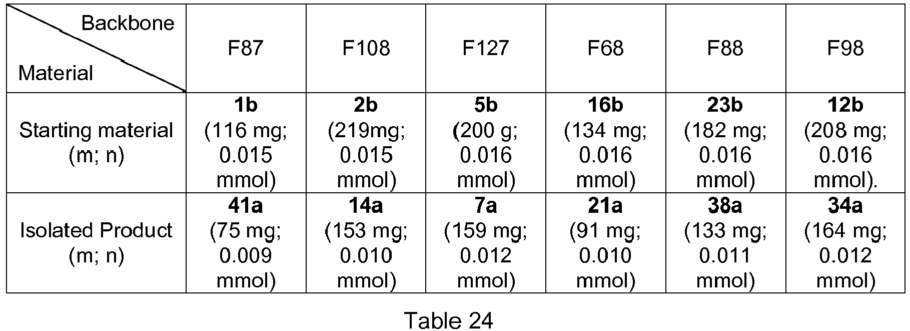

### Step2: Synthesis of bis-histidine substituted F127derivative VII.

Compound 7a (159 mg) is dissolved in 4 mL of a solution of dichloromethane. TFA is then introduced dropwise (VWR, 1 mL). The solution is degassed with argon and then stirred for 2h. Mixture is then concentrated under vacuum, and co-evaporated twice with DCM, three times with diethyl ether and dried one night under vacuum. The remaining residue is then resuspended in MilliQ water and introduced in a dialysis bag of 3.5 kDa cutoff (Spectrum Labs Europe). The dialysis is performed against 4 liters of double-distilled water with 5 medium-changes (2 hours, 8 hours, 24 hours, 36 hours and 48 hours). Final product is then recovered after lyophilisation (160 mg).
Following Table 25: deprotection of histidine step assessment: this table presents number and quantities of starting materials and name and quantity of the products bearing deprotected histidine moieties in which m represents the mass of the compound while n the number of moles.

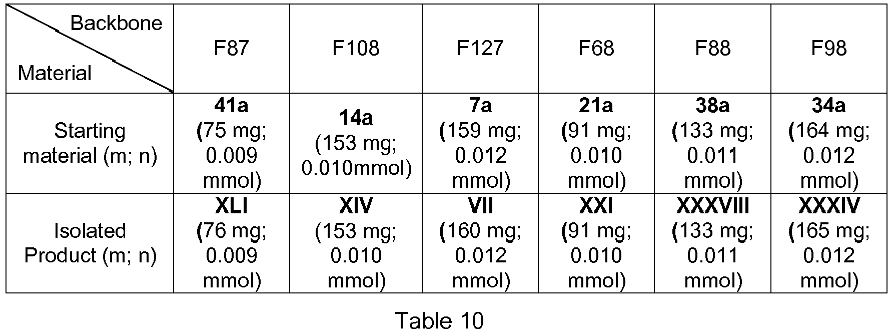

### Example 8: Synthesis of cationic poloxamer-coated magnetic nanoparticles.

Some of the cationic poloxamers derivatives described in this invention have been used to coat negatively or positively charged iron-based magnetic nanoparticles. Thus after the initial NPs synthesis several coating strategies have been envisioned. All of them involved the resuspension of a magnetic fluid (aqueous or ethylic suspension of NPs) in an aqueous polymeric suspension. We observed during initial trials huge differences in stabilizing behavior between the cationic poloxamers, depending on three mains factors:
- Cationic end group selection
- Nature of the polymeric backbone
- Concentration of the cationic poloxamer solution.

Based on these observations, several experimental strategies have been attempted in parallel to figure out what is the best way to get colloidally stable magnetic NPs coated with cationic poloxamers. So after the synthesis of the magnetic core by a well described and known in the art coprecipitation strategy (any other synthesis procedure can also be considered), the polymeric coating has been introduced using different pathways, depending on the concentration of cationic poloxamer solution used. We can sort these pathways in four categories as "diluted" "moderate concentration" "concentrated" and "highly concentrated" that corresponds to aqueous cationic poloxamer coating solutions being equal to respectively at 0.25, 1, 5 and 10% w/v, while the iron content is fixed each time at 0.5 mg of iron per mL of polymeric aqueous solution.

### Step 1: Synthesis of hydrophobically coated iron-based magnetic core.

The iron based magnetic core is obtained via a classical co-precipitation strategy (Lu, A.H. et al. Angew. Chem. Int. Ed. 2007, 46:1222-44; Huber, D.L. Small 2005, 1: 482-501). Using Iron (III) chloride hexahydrate (Sigma) and Iron (II) chloride tetrahydrate (Sigma) both dissolved in MilliQ water. After filtration, precipitation is carried out under an argon atmosphere upon mechanical stirring. NH₄OH (TCI) has been used to precipitate iron oxide. The hydrophobic precoating is then introduced at a temperature of 80°C, while stirring is maintained for 2 hours. After cooling to RT, the reaction is then transferred in an Erlenmeyer and is magnetically decanted over 12 hours. The supernatant is removed and the remaining solid is washed extensively with absolute ethanol (4 portions of 30 mL), and use as it in the next step, suspended in 20 mL of ethanol noted Solution A.

### Step 2: Coating of the magnetic core using an aqueous diluted solution of cationic poloxamer (0.25% w/v).

An aqueous solution of the cationic poloxamer of interest is prepared by suspending the polymer in MilliQ water at a concentration of 100 mg/mL (10% w/v). To help dissolution and prevent gelation, the polymer once suspended is kept at 4 °C for 12 hours while being stirred regularly. After complete dissolution, the solution is filtered on a 0.22 µm membrane, providing what we will call after the mother solution.

### Step 3.1: Coating of the magnetic core using an aqueous diluted solution of cationic poloxamer (0.25% w/v).

To work using the "diluted" conditions, 1mL of the mother solution is diluted with 37 mL of MilliQ water to give a Solution noted B. For the coating procedure, solution A is titrated using a standard colorimetric method (Jiang C et al.; J Magn Magn Mater. 2017, 439:126-134) providing the total iron concentration of the Oleic-acid coated magnetic core. Then a volume of solution A containing 0.5 mg of iron is magnetically decanted for 12 hours on magnetic rack (OZ Biosciences, Marseille, France). After removal of the supernatant, the remaining solid is then resuspended in 1 mL of solution B and vortexed intensively for 5 minutes. The resulting suspension is then sonicated (Branson apparatus, Power output 3, 70% duty cycles, 10 minutes sonication) before being characterized by DLS.

### Step 3.2: Coating of the magnetic core using a moderately concentrated solution of cationic poloxamer (1% w/v).

Previous steps 1 & 2 are repeated, and solution A is titrated as before. Meanwhile 100µL of polymeric aqueous mother solution is diluted in 900 µL of MilliQ water providing solution C (in which cationic poloxamer concentration equals 1% w/v). Then a volume of solution A containing 0.5 mg of iron is magnetically decanted for 12 hours on magnetic rack (OZ Biosciences, Marseille, France). After removal of the supernatant, the remaining solid is then resuspended in 1 mL of solution C and vortexed intensively for 5 minutes. The resulting suspension is then sonicated (Branson apparatus, Power output 3, 70% duty cycles, 10 minutes sonication) before being characterized by DLS.

### Step 3.3: Coating of the magnetic core using a concentrated solution of cationic poloxamer (5% w/v).

Previous steps 1, 2 and 3.2 are repeated, and solution A is titrated as before. Meanwhile 500µL of polymeric aqueous mother solution is diluted in 500 µL of MilliQ water providing solution D (in which cationic poloxamer concentration equals 5% w/v). Then the NPs resuspended in a 1% w/v cationic poloxamer (see Step 3.2.) are again magnetically decanted for 12 hours on a magnetic rack. After removal of the supernatant, the remaining solid is then resuspended in 1 mL of solution D and vortexed intensively for 5 minutes. The resulting suspension is then sonicated (Branson apparatus, power output 4, 80% duty cycles, 10 minutes sonication). After cooling to room temperature, a second sonication cycle is performed before characterizing the NPs by DLS.

### Step 3.4: Coating of the magnetic core using a highly concentrated solution of cationic poloxamer (10% w/v).

Previous steps 1, 2, 3.2 and 3.3 are repeated, and solution A is titrated as before. Then the NPs resuspended in a 5% w/v cationic poloxamer (see Step 3.3.) are again magnetically decanted for 12 hours on a magnetic rack. After removal of the supernatant, the remaining solid is then resuspended in 1 mL of cationic poloxamer mother solution and vortexed intensively for 5 minutes. The resulting suspension is then sonicated (Branson apparatus, power output 4, 80% duty cycles, 10 minutes sonication). After cooling to room temperature a second sonication cycle is performed, then a third one, before characterizing the NPs by DLS.

### Biological evaluation of the modified cationic poloxamers

### Materials & methods for the transduction experiments

### Cationic poloxamer formulation in water

An aqueous solution of the cationic poloxamer of interest is prepared by suspending the polymer in MilliQ water at a concentration of 100 mg/mL (10% w/v). To help dissolution and prevent gelation, the polymer once suspended is kept at 4 °C for 12 hours while being stirred regularly. After complete dissolution, the solution is filtered on a 0.22 µm membrane, and is ready to be used in transduction.

For adenoviruses or AAV-mediated experiments, 250 mg/mL (25%w/v) cationic poloxamers solutions have been prepared, following the same general guidelines to preserve a liquid form of the mixture.

### Cells and virus

Human cervical carcinoma (HeLa), Human Embryonic Kidney (HEK-293T), Adult Mouse Hypothalamic cell line (CLU-500), Mouse fibroblasts (NIH-3T3), immortalized murine microglial cell line (BV2), and Rat glioma (C6) cell lines were cultured in Dulbecco's Modified Eagle Medium (DMEM, Lonza, Walkersville, MD, USA) supplemented with 10 % Foetal Bovine Serum (FBD, St. Louis, MO, USA), 2mM final L-Glutamine, 100 units/ml penicillin and 100 µ/ml streptomycin (Lonza, Walkersville, MD, USA). Human immortalized Jurkat T-cells were cultured in Roswell Park Memorial Institute medium (RPMI, Lonza, Walkersville, MD, USA) supplemented with 10 % Foetal Bovine Serum (FBD, St. Louis, MO, USA), 2mM final L-Glutamine, 100 units/ml penicillin and 100 µ/ml streptomycin (Lonza, Walkersville, MD, USA). CD34+ KG-1a cell line was cultured Iscove's Modified Dulbecco's Medium (IMDM, Lonza, Walkersville, MD, USA) supplemented with 20 % Foetal Bovine Serum. Primary CD34+ stem cells isolated from cord blood and were cultured in P6-well plates and all cell lines were cultured in 75 cm² flasks (Sarstedt AG & Co., Germany) at 37°C in a humidified incubator (Sanyo, Tokyo, Japan) with 5% CO2 atmosphere, and trypsinized at 80% confluency in order to maintain an exponential division rate before transduction assays. Transduction experiments were performed in 24 well-plate (Sarstedt AG & Co., Germany).

### Preparation of the Lentivirus/Cationic poloxamers solution for infection

HIV-1-derived vector coding for the green fluorescent protein (GFP) reporter gene under the control of a SFFV promoter (HIV-SFFV-GFP) was produced at the facility of SFR Biosciences (UMS3444/CNRS, US8/INSERM, ENS de Lyon, UCBL, France). Viral suspensions were collected from cell supernatants and aliquoted without any concentration step before conservation at -150°C (Sanyo, Tokyo, Japan). Previous to each experiment, virus stock was thawed and diluted at the desired Multiplicity of Infection (MOI) in 50 µL of complete medium. After addition of the cationic poloxamer, suspension was mixed by tube inversion and directly added in a dropwise manner onto cells. Cells were then incubated for 72H under classic condition until evaluation of experiment.

### Adenovirus and Adeno Associated Virus infection

Type 5 replication-deficient Adenovirus encoding GFP (Ad-GFP) and AAV serotype A virus, both under the control of a cytomegalovirus (CMV) promoter were purchased from Vector Biolabs. Ad-GFP aliquots of 1x10¹⁰ IFU/ml viral stock corresponding to 5x10¹¹ VP/ml were diluted extemporaneously in medium without any supplement in order to reach the desired MOI. AAV-1 were diluted in medium without supplement for a final MOI of 100.000 genome copies per cell. When needed, cationic poloxamers mixed with viral particles and added in a dropwise manner onto cells. Cells were then incubated for 72H under classic condition until evaluation of experiment.

### Flow Cytometry.

Cells were washed two times with phosphate buffered saline without calcium and magnesium (PBS, Lonza, Walkersville, MD, USA) and adherent cells were detached with a Trypsin/EDTA 0.2 % solution (Lonza, Walkersville, MD, USA) before being fixed in 200 µL of 4 % PFA (Sigma Aldrich). Cells were then analysed for % of GFP positive cells and fluorescence intensity by flow cytometry using a CytoFlex Flow Cytometer (Beckman Coulter, Miami, FL).

### Evaluation in transduction experiments of the use of modified cationic poloxamers as chemical transduction enhancers using Lentiviruses.

As disclosed herein, the main purpose of this invention is to develop a new method with chemical agents able to enhance efficiently and reliably the infective behavior of viruses. The first part of the results focuses on the use of lentivirus as viral vector, as it has been described as potent candidate for cell therapy clinical studies. For this evaluation we used mouse fibroblast cell lines, NIH-3T3; the cationic poloxamers described in the examples have been associated with HIV-1-derived vector coding for the green fluorescent protein (GFP) reporter gene. This first evaluation described modified cationic poloxamers derived from F87, F127 and F108.

NIH-3T3 cell line was infected with Lentivirus (MOI 1) in presence or not of various doses of cationic poloxamers. After 72 h incubation, % of GFP positive cells (A) and mean intensity (B) of transduced cells were evaluated by flow cytometry. Results are described in Figure XI.

The biological evaluation of cationic poloxamers as transduction enhancers highlighted well their propensity to act positively in such experiments. Compounds **I, 2b, II &VI,** show a net increase in the number of cells infected compared with the viral vector used without adjuvant at the same MOI, with a maximum 6-fold increase when using 10 µL of compound **II.** This increase is also visible to a lesser degree when comparing the mean intensities of the different experiments.

For the compound showing a positive effect, clear dose dependence is noticeable, with a dramatic increase of the numbers of infected cells related to an increasing amount of cationic poloxamer. This dose effect is less visible when considering the mean of intensity, a clear increase is observed only when compound **2b & VI** are chosen.

On the other hand, compound **III** showed no positive effect on the transduction while used in the same experimental conditions. The number of infected cells remains at the same level than with the lentivirus alone, whatever the cationic poloxamer dose employed. It is however important to note that compound **III** does not inhibit the behavior of the virus, and does not affect its infecting skills.

The difference of behavior between compounds **III** and **VI,** both based on F127 backbone, is a first proof of the tremendous importance of the ending group nature on the transduction efficiency.

A second set of potent adjuvants has been evaluated following the same experimental plan than previously. Results are summarized in Figure XII.

This evaluation of the different cationic poloxamers candidates gives similar results to the previous one. It indeed seems that some of the modifications have clearly a positive impact on the transduction efficiency while others just do not lead to any improvement.

In case of tetrazole-based compounds **4b and IV,** only the IV leads to a net improvement of the transduction efficiency, both in terms of number of infected cells and of mean of GFP intensity. The dose response is also clearly visible, highlighting the positive impact of the adjuvant. In the same time, the use of a non-substituted tetrazole ending groups to modify the cationic poloxamer did not bring the same level of enhancement. Indeed, when **4b** is used as transduction enhancer, the number of cells transduced remained really close to the level reached with the lentivirus alone.

Imidazole-based compounds **5c & V** both show a positive effect in transduction associated with a lentivirus. In both cases the number of cells infected by the virus is dependent to the amount of modified cationic poloxamer used. However, the improvement is much more pronounced with compound **V,** that shows around four times more transduced cells than the virus alone. In this case also, the methylation of the heteroatomic ring lead to a better behavior as adjuvant, with compound **V** being more efficient than its counterpart **5c.**

### Importance of the modification: comparison between unmodified poloxamer and cationic poloxamer as adjuvant in lentivirus-mediated transduction experiment

The innovative feature of this invention stands on the cationic modification of the ending groups of poloxamers to enhance their adjuvant skills in transduction experiments. The biological activity of the modified cationic poloxamers with their non-modified counterpart were then compared. Accordingly, we used compounds **2b & II** as modified cationic poloxamers and compared them with Pluronic F108, associated or not with polybrene, following a well-described procedure.

Two cell lines were tested, namely Human Embryonic Kidney (HEK-293T) and immortalized murine microglial cell line (BV2). The cationic poloxamers have been associated with HIV-1-derived vector coding for the green fluorescent protein (GFP) reporter gene. Results are depicted in Figure XIII.

Results pointed out that the three cationic poloxamers efficiently enhance the transduction in both cell lines. F108 in combination with the cationic polymer, polybrene doubles the number of BV2 cells infected by the virus compared with the virus alone. More interestingly, the introduction of cationic endgroups into the F108 backbone has a net positive effect on the number of positive GFP cells, which goes from 18% when F108 + polybrene is used as adjuvant to 26% for compounds **2b & II.** In another hand, the mean of fluorescence intensity remained unaffected whatever the polymer used. In the case of HEK-293T cells, the positive effect of the use of cationic poloxamer on the transduction is also clearly noticeable. The unmodified and modified cationic poloxamers provide the same enhancement for the number of infected cells. This number goes from around 40% when the LV is used alone to around 60% when a cationic poloxamer is used as enhancer. However, the mean of fluorescence intensity is clearly enhanced by the introduction of cationic endgroups on the adjuvant. Indeed, with both **2b and II,** this value is higher compared with the F108, gaining almost one third after the cationic modifications. In the end, these results clearly demonstrate the benefits of cationic modification of the poloxamer backbone in different parameters related to global efficiency of the procedure.

Moreover, it is clear from the previous evaluations, that the nature of the cationic function introduced has a vital importance for the success of the transduction experiment enhancement. Consequently, we have compared the transduction efficiency of different cationic poloxamers based on a F108 backbone, but modified with different polymeric functions. Compounds, 2b, 4b and IV were evaluated in parallel on human Jurkat T-cells. The cationic poloxamers have been associated with HIV-1-derived vector coding for the green fluorescent protein (GFP) reporter gene. As before the F108 has been used as control. Results are depicted in Figure XIV.

The result confirmed that compound **2b** once again provides better results than its non-modified counterpart F108. Indeed, while both cationic poloxamers induces 20% more cells to be infected by the viral vector, the cationic modification provokes an increase of the mean of intensity of 20% versus the F108. On the other hand, results with compounds **4b** & **IV** are less efficient. On Jurkat cells, they both induce a slight increase in the number of transduced cells compared with the viral vector used alone and no significant effect in comparison to virus alone, were observed in terms of mean of intensity. More importantly, the results observed with the compounds 4b and IV were lower than with the non-modified cationic poloxamer highlighting the critical needs to design a suitable cationic modification.

By confirming that the behavior of the cationic poloxamer in transduction experiment is dependent on the nature of the cationic endgroup introduced, this experiment supports the fact that the careful choice of the modification is the key of the success in such procedure. Indeed, introducing a cationic group is not sufficient to get transduction improvement over the virus alone and furthermore against the same reaction adjuvanted by a non-modified poloxamer.

### Transduction optimization: cell screening and dose responses

In order to achieve optimal transduction efficiency, we have monitored various parameters such as cationic poloxamer quantity, virus titer, type of cells etc. The cationic poloxamer dose response study has been carried out using KG-1a cell line, which are immortalized human bone marrow acute myelogenous leukaemia cells, known to be very difficult to infect using classical lentiviruses-mediated transduction procedures. Various doses of cationic poloxamers have been associated with HIV-1-derived vector coding for the green fluorescent protein (GFP) reporter gene. Results are depicted in Figure XV.

The results highlight again the positive effect of the use of compounds **2b & II** in this kind of transduction procedure. Indeed, while transduction mediated with the lentivirus alone fails to give more than 2% of infected cells at MOI 2 after 72 hours of incubation, the use of cationic poloxamers increases this level to values close to 15% at the same MOI. Results are really close between **2b & II,** with in both cases a clear dose response effect while increasing the amount of adjuvant added.

This positive effect is confirmed by the analysis of the mean intensity of fluorescence. With both compounds this value increases spectacularly compared with the use of the viral vector alone. The best observed result is obtained when 10µL of compound **2b** is used as additive, with a 9-fold gain compared to the control. Dose response profile are slightly different between **2b & II,** as the first reached its maximum at a value of 10 µL whereas compound **II** induces a constant increase of the mean intensity between 2µL and 20µL, not reaching a dose plateau like **2b.**

In conclusion both cationic poloxamers showed a dose-dependent positive effect on the transduction mediated by lentiviruses. Despite their structural similarity, compounds **2b & II** present slight differences in behavior depending on the dose added, which emphasizes again the importance of the choice of the cationic function on the global efficiency of the transduction procedure.

The next experiment had for objective to demonstrate the ability of cationic poloxamer to enhance the efficiency of lentivirus-mediated transduction procedure using a large variety of cell types, and thus to demonstrate the broad-range character of the present invention.

Compound **2b** has been tested on 6 different cell lines which are Human cervical carcinoma (HeLa), Adult Mouse Hypothalamic cell line (CLU-500), immortalized murine microglial cell line (BV2), Rat glioma (C6) cell lines, human immortalized Jurkat T-cells and CD34+ KG-1a human bone marrow acute myelogenous leukaemia cell line. HIV-1-derived vector coding for the green fluorescent protein (GFP) reporter gene has been used as viral vector, optimized MOI for each cell line has been selected to emphasize on the cationic poloxamer effect. Results are depicted in Figure XVI.

This study on a variety of cells clearly demonstrates the consistency of the cationic poloxamer used as adjuvant in lentivirus-mediated transduction experiment. Indeed, whatever the cell line tested, introduction of compound 2b always lead to a clear increase of the number of cells infected. This gain varies depending on the cell line used, ranging from about 10% increase of number of infected cells compared with virus alone (Jurkat) to 650% benefit with KG1-a. The homogeneity of these impressive results can also be observed when looking at the mean of the fluorescence intensity. With this readout as well, the use of the cationic poloxamers as adjuvant enhances notably the results obtained with the lentivirus alone in all cell lines tested, the enhancement being highly cell-dependent. Whereas the benefit of intensity is only of a factor 1.5 when considering BV-2 cell line, transduction of KG1-a using compound 2b is 2.5-fold more intense compared with the virus alone. In conclusion of these series of experiments, the benefits of the cationic modification of the poloxamer backbone as enhancer for transduction experiments mediated by lentivirus was clearly demonstrated. Cationic modification of the poloxamers evidently influence their adjuvant properties. It gives rise to a panel of adjuvants well-suited to the requirements meet in gene therapy applications.

Finally, the cationic poloxamers' efficiency to enhance lentiviral induced transduction was confirmed on primary human CD34+ stem cells issued from cord blood. A dose response study assessed the capacity of cationic poloxamers to infect primary human CD34+ cells with HIV-1-derived vector coding for the green fluorescent protein (GFP) reporter gene. Results are depicted in Figure XVII and show the positive effect of the use of compounds **2b & II** to raise transduction efficiency to 35% at MOI 5 after 72 hours incubation compared to the lentivirus alone that gives about 7% of infected cells at the same MOI of 5 after 72 hours of incubation.

As observed previously cationic poloxamers also succeeded in dramatically improving the mean intensity of fluorescence when compared with the use of the viral vector alone. The best observed result is obtained when 20µL of compound **2b** is used as additive, with a 5-fold gain compared to the control.

In conclusion these results demonstrate the capacity of the cationic poloxamers to enhance the transduction of primary CD34+ stem cells mediated by lentiviruses in a dose-dependent manner.

### Use of cationic modified poloxamers as adjuvant in Adenoviruses-mediated transduction experiments.

To extend the scope of the current invention, the effect of the cationic poloxamers as efficient adjuvant in adenovirus-mediated transduction experiment was also investigated. As described in the introduction, non-modified poloxamers have been used as adjuvant in adenovirus-mediated transduction experiments but required very high concentration. In these conditions, the polymer acts as a "pseudo-gel" agent that reduces the diffusion of the viral particles in the biological medium. The main consequence is that as the viruses diffuses less and it is more likely to stay longer in contact with the cells to infect. This prolonged contact increases mechanically virus uptake and thus the efficiency of the whole procedure. It was possible to formulate cationic poloxamer **2b** at a 25% w/v concentration which is near its gelation point, demonstrating that the cationic modifications did not inhibit the "gel" behavior observed with the corresponding non modified poloxamer. The main goal of this experiment is to check the pseudo-gel efficacy of the modified cationic poloxamers and also, to monitor if the cationic modifications bring a supplement of efficiency for the adenoviral-mediated transduction procedure, as it was the case with lentiviruses.

To do so, C6 and HEK-293 cell lines were transduced with adenovirus encoding for GFP (AdGFP) with a MOI of 5 in presence or not of commercially available F108 and its cationic counterpart **2b** at a 7.5% w/v final concentration. The results of this experiment are depicted in Figure XVIII.

This experiment clearly demonstrates the benefit of using a modified cationic poloxamer as transduction adjuvant associated with adenoviruses. Indeed, on either HEK-293 or C6 cells, the adenovirus used alone failed to infect 5% of the cells. On the other hand, the cationic modifications lead to a significant enhancement of the adenovirus transduction efficiency. Indeed, when compound **2b** is used against its unmodified counterpart, the number of infected cells raises from 46% to 55% on C6 cells and from 32 to 46% on HEK293 cells. The positive effect of the cationic modification is even more impressive when considering the mean of fluorescence intensity with almost a 2-fold increase with compound **2b** on both cell lines. This experiment demonstrates that cationic modification of poloxamer opens a wide-range of applications for virus-mediated transduction experiment and that the observed improvement is not limited to lentiviruses.

### Use of cationic poloxamers as adjuvant in AAV-mediated transduction experiments.

To extend further the scope of this invention, the effect of the cationic poloxamers were also tested as efficient adjuvant in AAV-mediated transduction experiment. Compound **II** at a final concentration of 15% w/v has been associated with AAV(GFP) and tested on HEK-293 cells. The results of this experiment are resumed in Figure XIX:
In this procedure again, the use of modified cationic poloxamer proved to be a real source of enhancement of the efficiency of the transduction procedure. The addition of 15% w/v of compound **II** allows AAV to infect 57% of cells while AAV used alone infects only 12%. The improvement is less important when considering the mean of intensity, with nonetheless a net gain of 5% compared with virus alone. In the end, this experiment demonstrated that the cationic poloxamers are also valuable and efficient as adjuvant in AAV-mediated transduction experiment.

### Cationic poloxamers and magnetic nanoparticles formulation: DLS Characterization of the cationic poloxamer-coated MNPs

As mentioned previously, magnetic nanoparticles (MNPs) have been demonstrated as powerful to boost, accelerate and synchronize viral infection. Consequently, it could have been foreseen that the combination of magnetic nanoparticles and unmodified poloxamer lead a strong synergistic effect. Unfortunately, the non-ionic nature of unmodified poloxamer lead to nanoparticles destabilization or precipitation which hampered their use as coating or formulating agent of nanoparticles.

To investigate the benefits of the poloxamer cationic modification on the magnetic nanoparticle's formulation and stabilization, we focused first on DLS (Dynamic Light Scattering) to measure the size and the charge of the obtained nanoparticles. Size and net charge (zeta potential) measurements are nowadays key parameters to check when focusing on MNPs synthesis and characterization. Indeed, a stable colloidal suspension is mainly characterized by its propensity to maintain the magnetic objects in a non-aggregated state. In other words, it is critically important that the nanoparticles present in solution remain of nanometric sizes. This is even more important when this colloidal suspension is intended to have biological applications, as aggregated objects have proved in the past to have disastrous consequences on the cell's viability. The zeta potential is a direct electric consequence of the presence of charged chemical functions at the surface of the nanometric object. Thus, a suspension made of charged nanoparticles (either positively or negatively) is less likely to witness aggregation of its components, because of the electrostatic repulsions which limit the propensity of magnetic cores to stack into micrometric objects. Furthermore, a net positive charge will allow these beads to form stable complexes with negatively charged viruses (such as many Lentivirus or adenovirus), which appears to be a favorable step in a magnetically-driven transduction experiment.

The lack of chemically charged functions in the native unmodified poloxamer backbone forbids any repulsive interactions when associated with magnetic beads and leads to destabilization and/or aggregation of those nanoparticles. On the of goal of this present invention was to demonstrate that the cationic modified polymers are able to stabilize magnetic MNPs by providing to the nanometric objects synthesized, enough positive charges on its surface to create electrostatic repulsive interactions, to be able to maintain the colloidal suspension and to allow achieving a viral transduction enhancement. As a consequence, the resulting MNPs will cumulate several features useful for an efficient magnetic NPs-driven transduction experiment:
- ability to complex virus at the surface of the NPs, following Magnetofection™ requirements
- presence of cationic poloxamer in the complex to act as adjuvants during transduction
- stabilization of the magnetic NPs to avoid the formation of micrometric objects, detrimental for cell viability.

Following these guidelines several magnetic nanoparticles have been synthesized using a well-described co-precipitation strategy. The obtained iron core is mainly made of magnetite crystals, conferring the object strong magnetic properties. The strategy involved the use of a pre-coating molecule prior to the cationic poloxamer introduction. This pre-coating molecule must be charged, negatively or positively, in order to stabilize the magnetic core, and may also include hydrophobic areas, that will be useful to associate the cationic poloxamer to the surface of the NP through hydrophobic interactions. Examples of precoating include a wide array of well-known molecules such as dextran, starch, Zonyl FSA, Linoleic acid, oleic acid, silicium oxide polymer, polyethyleneimine, chitosan, Tween, Span. In the following study, it was chosen to use an oleic acid-based pre-coating.

Different unmodified or cationically modified poloxamers solutions have been used to resuspend this MNPs, while the quantity of iron has been kept constant.

### Coating of the magnetic core using aqueous diluted and concentrated solutions of cationic poloxamer.

The MNPs were first resuspended with 0.25% w/v of cationic polymer solution, then after another decantation, the MNPs were formulated within the corresponding 1% w/v cationic poloxamer solution. This intermediate dilution/decantation step appeared to be a reliable solution to avoid direct aggregation of the resulting MNPs. Only the NPs suspended in the 1% w/v solution were evaluated by DLS.

Abroad array of modified polymers was tested to figure out if other interactions such as (hydrophobic, pi/pi...) brought by the cationic endgroups could be advantageous over short (5 days) and long incubation time (1 month). The size measurements results are presented in the Table 26 below:

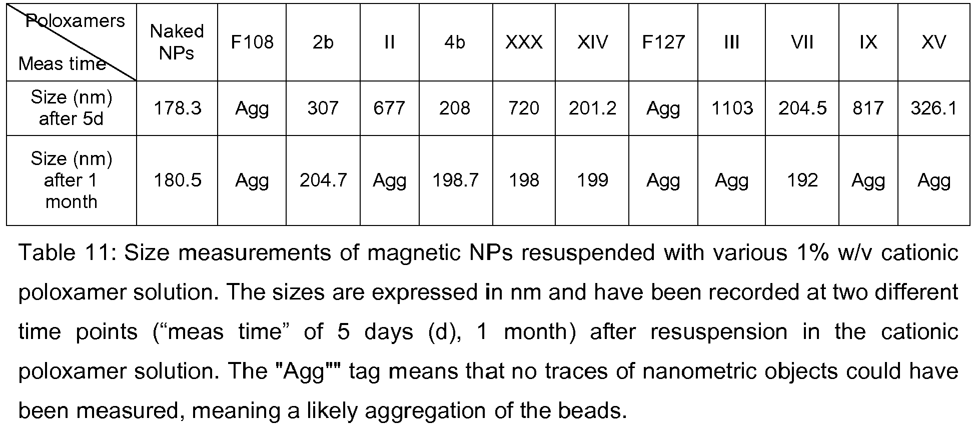

This experiment highlights the impossibility to stably formulate or coat magnetic NPs with a diluted solution of the native (non-ionic) or non-modified poloxamer. Indeed, it leads to the quick, systematic and irreversible aggregation of the beads, whatever the polymer chosen, either F108 or F127.

In the case of MNPs coated or formulated with cationic F108-derivatives (**2b, II, 4b, XXX and XIV**) all cationic poloxamers gave, after the resuspension, colloidally stable nanoparticles after 5 days. Amines (**2b**), triazole derivative (**4b**) and histidine (**XIV**) look the most promising candidates as stabilizing agents for MNPs leading to MNPs having sizes in the perfect range for transduction applications, *i.e.* between 200 and 300 nm. This result was confirmed after 1 month of production When compared with non-coated NPs, the introduction of cationic polymers at the surface of the NPs induces a small increase in the global size of the construct, with a distribution peak that remains thin after the redispersion/sonication procedure, highlighting the association between the cationic polymer and the bead surface.

However, and despite their cationic modifications, some cationic poloxamers such as the **II** presented a characteristic behavior of aggregation, which was not completed after 5 days (size of 677 nm), but was after one month. The MNPs formulated with various cationic poloxamer based on the F127 backbone, showed also potential as stabilizing agents for magnetic nanoparticles (see Table 29); one cationic poloxamers (**VII**) led to a stable MNPs formulation, even after 1 month.

In summary, while it was not possible to formulate stable MNPs coated with commercially available non-modified and non-ionic poloxamers, it was proved that the cationic functions introduced on the poloxamer endgroups is a necessary and mandatory condition for the stability of the corresponding coated MNPs. Moreover, it appears that depending on the property of the cationic endgroups, the stability of the MNPs is variable. Secondary interactions such as hydrogen bonding (compounds **2b**), pi/pi stacking (compounds **XXX)** or both (compounds **VII & 4b**) might be involved as well. In parallel of these measurements Zeta potential has been evaluated with the same compounds at the same time points, results are depicted in the following Table 27:

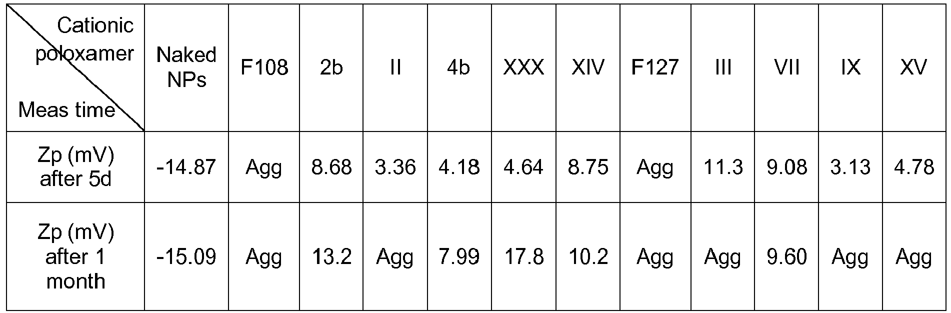

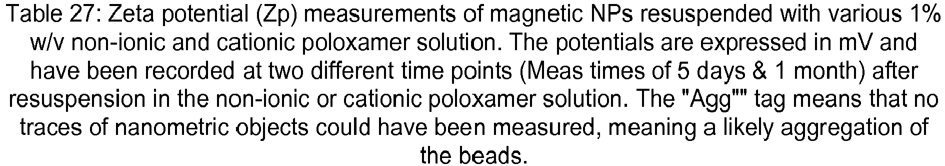

The results presented in this table correlate extremely well with the previous size measurements. Commercially available native and non-ionic poloxamer F108 and F127 when used as coating agents, mask some of the negative charges of the original MNPs, decrease their stabilization through electrostatic interactions and favor their aggregation. On the other hand, cationic poloxamers, all provide to the resulting nanoparticles a positive surface charge which clearly helps the colloidal stabilization over time, highlighted by the great behavior of compounds **2b, 4b, XIV, VII** even one month after the resuspension procedure. These experiments validate the fact that the introduction of cationic endgroups into the structure of poloxamers allowed to stabilize NPs coated with these modified polymers, contrary to their native counterparts.

Using this strategy, we further extend the coating and formulation of MNPs with higher concentration of cationic poloxamer (5% and 10% w/v). We selected for this study compounds **2b, 4b** and **XXX** derived from the F108 backbone, and compound **VII** derived from the backbone of the F127. To accomplish this, a resuspension procedure which involves intermediate stage of suspension/decantation with less concentrated cationic poloxamer solution has been used.

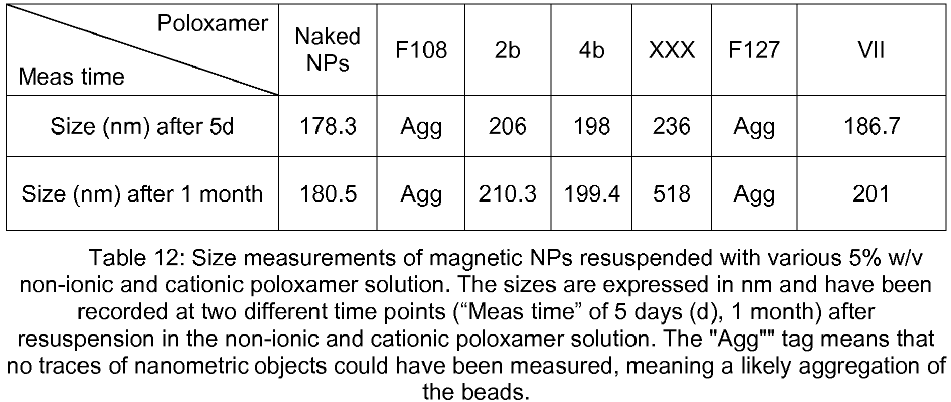

As before, the unmodified and neutral poloxamers (F108 and F127) have led to an instantaneous aggregation of the MNPs whereas the cationic poloxamers were able to coat in a stable manner MNPs at this higher concentration. On the other hand, the four selected compounds led to the achievement of stable beads after 5 days, having size ranging around 200 nm suitable with for transduction applications and minimized cell-toxicity. The nature of the chosen compounds does not seem to have an influence on the recorded size, with the exception of beads derived from compounds **XXX,** which looked slightly bigger than its relative. The 1-month stability study displayed no clear differences; most of the NPs keeping equivalent sizes compared with the sizes recorded 5 days after the resuspension. Here again, the beads derived from compound **XXX** showed a difference, with size ranging above 500 nm. This net increase in size might be a sign of preliminary stacking leading to aggregation, even if no sign of such phenomena is evident at this time point. As a conclusion, here again, the electrostatic repulsive interactions embedded by the cationic modifications of these poloxamers allowed the formation of stable suspension of magnetic NPs, even if the concentration of the cationic poloxamer solution used for resuspension is increased. The corresponding zeta potential values recorded at the same time points are presented in the Table 29 below.

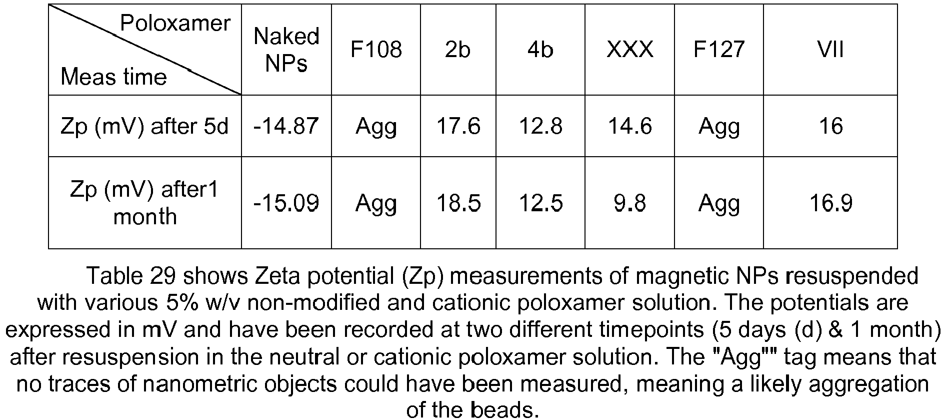

Zeta potential measurements correlates perfectly with the results observed for sizes. All the nanoparticles exhibit high positive charges after formulation with the cationic poloxamers, without any significant changes after 5 days or one month. Only the beads resulting from polymer **XXX** showed a slight decrease of the Zeta potential, possibly due to the increase in size. These data confirmed that concentrated solutions of cationic poloxamers might be suitable for the resuspension of decanted magnetic NPs. The cationic modifications at this concentration still allowed electrostatic repulsive interactions able to stabilize the colloidal nanostructure.

The last part of this detailed study, concerned the resuspension of the magnetic cores into 10% w/v cationic poloxamers solutions. This concentration is the preferred one to get the best results in lentivirus-mediated transduction experiments using cationic poloxamers as adjuvant. Having beads stably suspended at such concentrations might emphasize the adjuvant effect of the cationic poloxamer. We selected the same compounds (**2b, 4b, VII, XXX)** than previously to resuspend the beads. Here again a sequential decantation/resuspension procedure has been carried out to avoid direct aggregation of the beads. This time, the only chosen time point is 14 days. The size results are summarized into the Table 30 below:

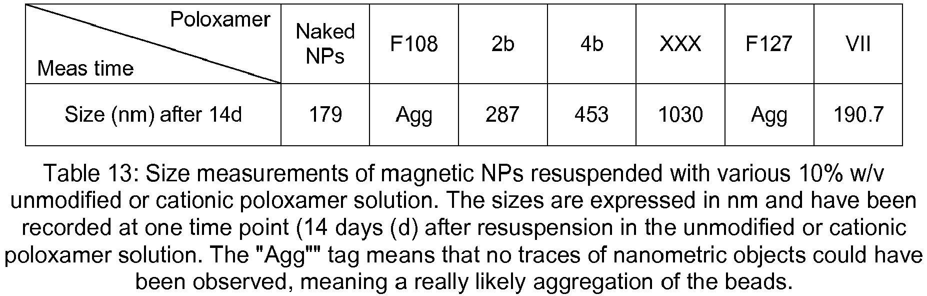

At this concentration, once again commercially available unmodified poloxamer 10%w/v solutions have not been able to resuspend efficiently the magnetic nanoparticles; in contrast the cationic modifications succeeded in stabilizing the MNPs. The compounds 2b and VII still impressively stabilize the colloid with only a slight increase in size of the beads coated by 2b and VII and without any sign of aggregation at this time. Those 2b and VII cationic poloxamers are clearly the best candidates for resuspension at this concentration, that is emphasized by the analysis of the Zeta potentials recorded in the same conditions. Nonetheless, some cationic poloxamers such as compound XXX did not allowed the achievement of stable nanometric NPs solution and induced stacking of the beads probably due to stronger Pi/pi interactions at this concentration. The results with compound 4b showed that a size of 453 nm that clearly indicates the beginning of an aggregating behavior, which is not completed after 14 days.

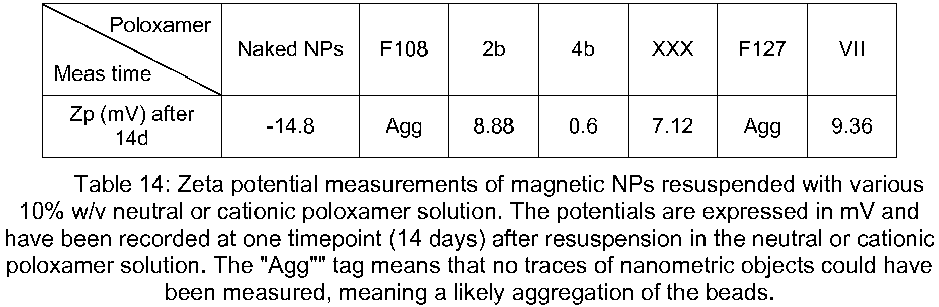

This last set of data is in total accordance with the previous ones. The compounds able to maintain a positive charge at the surface of the particles (**2b, VII)** are less likely to induce aggregation during the resuspension step. In contrary, the beads coated with **4b** became almost neutral with time. Compound **XXX** looks like an exception from this aspect, as its positive charges seem to not counterbalance the aggregation probably caused by Pi stacking of the triphenylphosphonium moieties. This highlights the fact that secondary interactions might have positive impact on the stabilization of the particles if they are correctly balanced and do not become a factor of stacking or aggregation.

As a conclusion, we clearly demonstrated the positive impact of the cationic modification of the poloxamers on the stabilization of resuspended magnetic nanoparticles. In opposition to commercially available neutral poloxamer, some modified cationic poloxamers create positive charge on the surface of the beads to ensure a colloidal stabilization through electrostatic repulsive interactions. With a careful choice of the chemical function and an optimized decantation/suspension strategy, MNPs with appropriated iron/cationic poloxamer content have been stably obtained. They have been tested in a magnetically driven transduction experiment mediated by lentivirus.

### Biological evaluation of the modified magnetic nanoparticles coated with cationic poloxamers

This invention clearly highlighted the reliable benefits of using cationic poloxamers to ensure a colloidal stabilization of magnetic nanoparticles. Next, the purpose was to check if such MNPs are able to associate the positive adjuvant effect described earlier herein with the propensity of cationic nanoparticles to enhance the efficiency of lentivirus-mediated transduction experiment. We have thus compared the transduction efficiency on KG1a cell line, of a lentiviral vector associated with either:
- ViroMag nanoparticles, from OZ Biosciences (Marseille, France) which are a longtime reference in enhancing lentivirus-mediated transduction,
- Cationic poloxamer **2b**, which has proved earlier in this document to be efficient as adjuvant in transduction experiment,
- ViroMag nanoparticles coated with cationic poloxamer **2b,** following the strategy developed in the present document.

KG1a cells were transduced with Lentivirus encoding for GFP with a MOI of 5 associated with ViroMag magnetic nanoparticles (VM), cationic poloxamer **2b** or ViroMag NPs coated with a 5% w/v solution of cationic poloxamer **2b** (VM+**2b**). After 72 h incubation, % of GFP positive cells (A) and mean intensity (B) of genetically modified cells were evaluated by flow cytometry. The results are depicted in Figure XX:
The results showed the benefits of the cationic poloxamer. The ViroMag MNPS were successful at enhancing the transduction as expected and published, as it allows to double the number of cells infected by the lentivirus. Compound **2b,** as described earlier allowed also to boost transduction efficiency, as the number of cells infected when it is used as adjuvant reached almost 40 %, with a mean of intensity 1.5-fold higher than the LV alone. The combination of cationic poloxamer **2b** with ViroMag led the highest efficiency. The synergistic effect brought by the cationic poloxamer and the MNPs is demonstrated. Indeed, **VM+2b** potentialized the effect of the adjuvant **2b** with the benefits of magnetically driven transduction experiment leading to higher number of cells transduced and higher mean of intensity. This experiment not only confirmed the benefits of introducing cationic functions on the endgroups of poloxamers to enhance their adjuvant behavior, but also proved that this cationic modification also allows to associate the intrinsic enhancing features of modified cationic poloxamers with the benefits of magnetically driven lentivirus transduction in only one formulation. This is of a critical importance to face today's emerging challenges of the use of virus mediated gene therapies or the use if virus to generate cell therapy product for clinical trials.

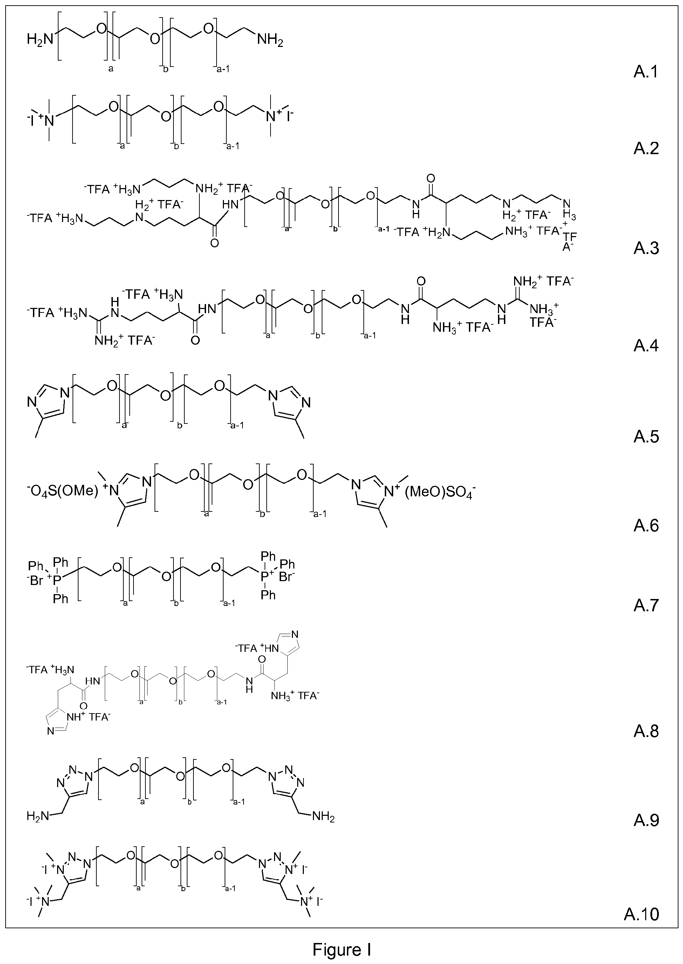

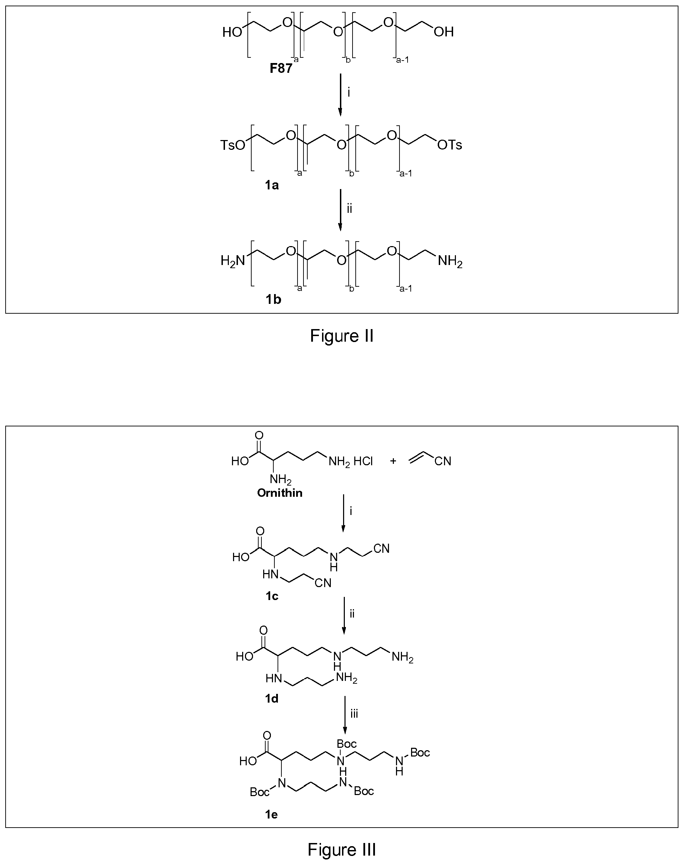

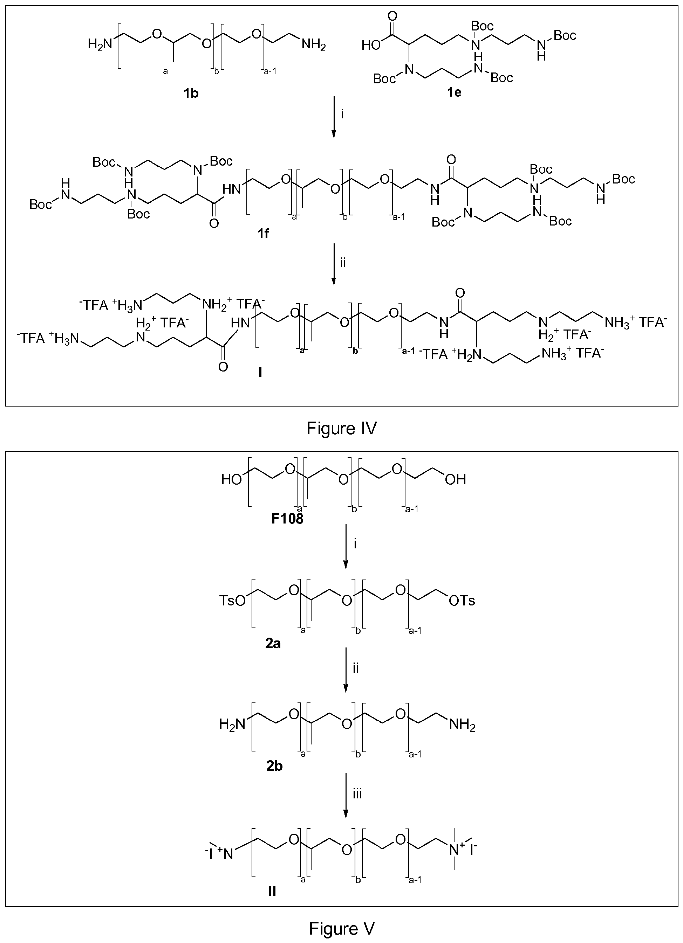

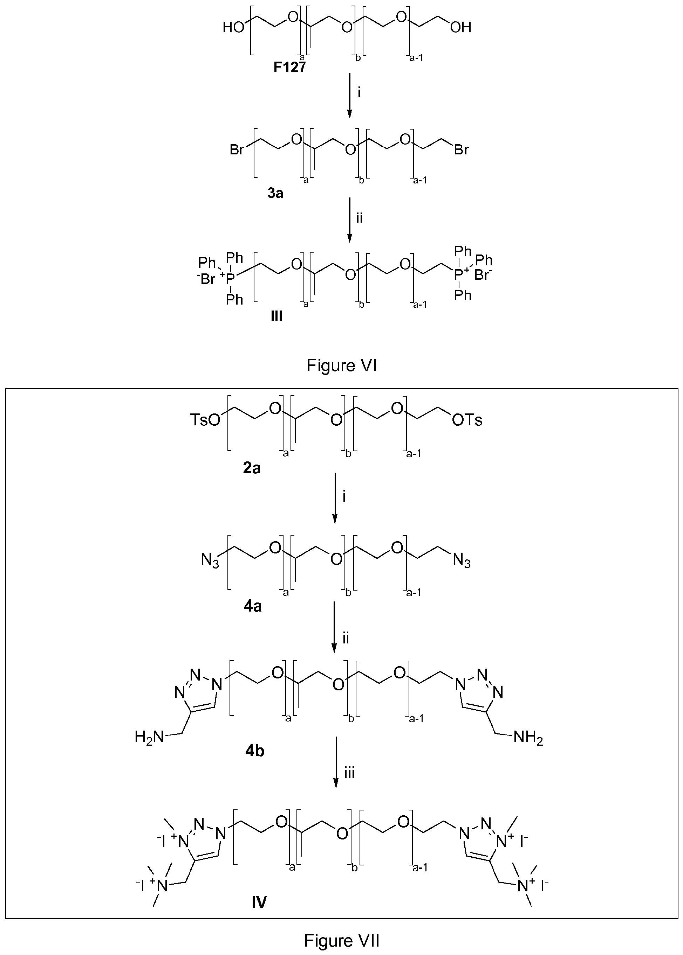

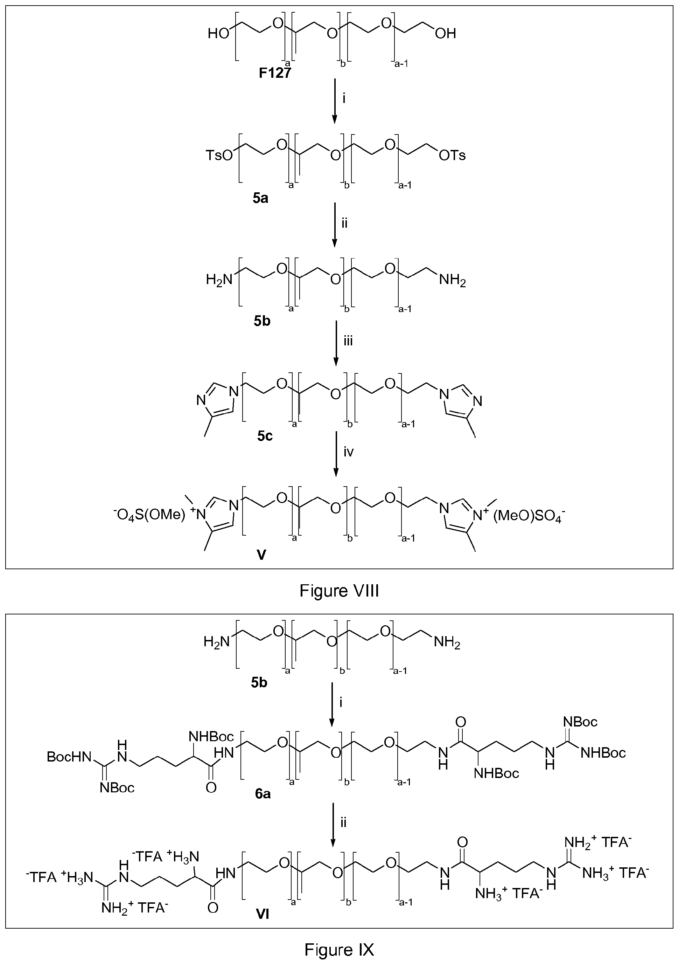

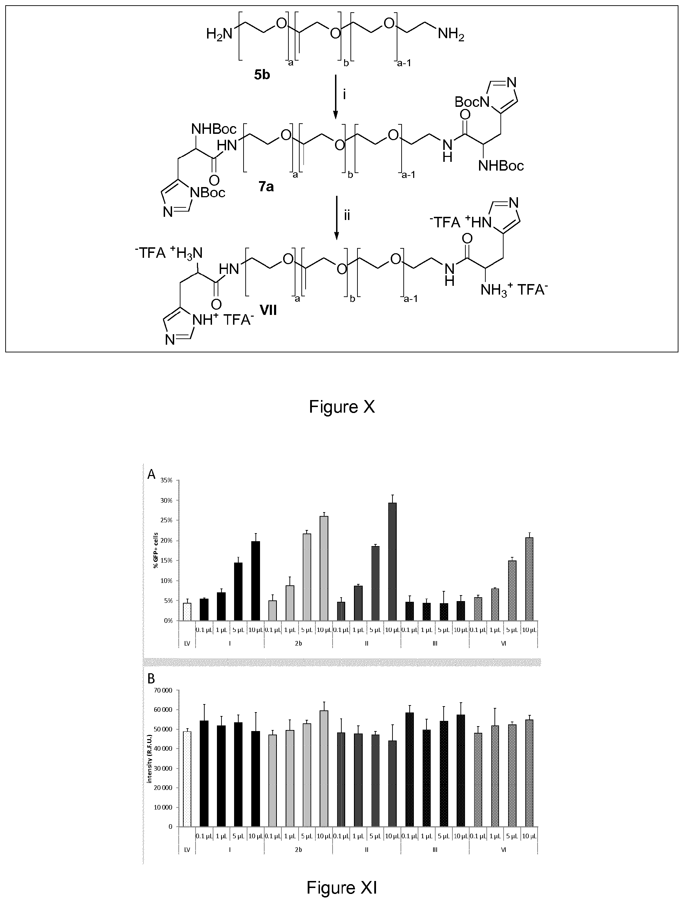

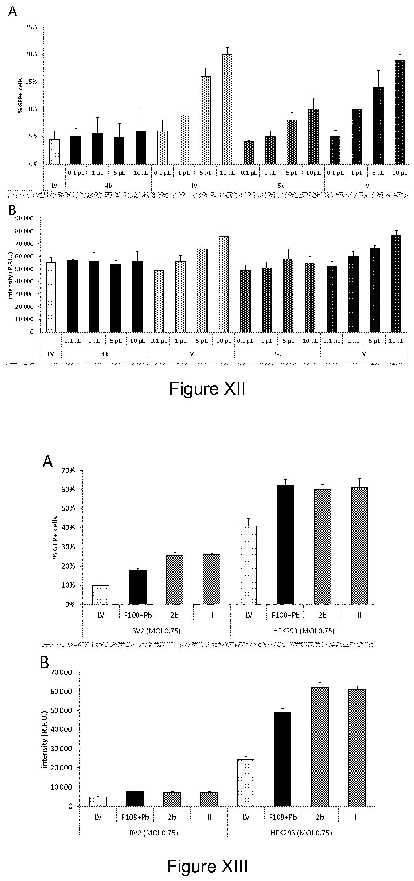

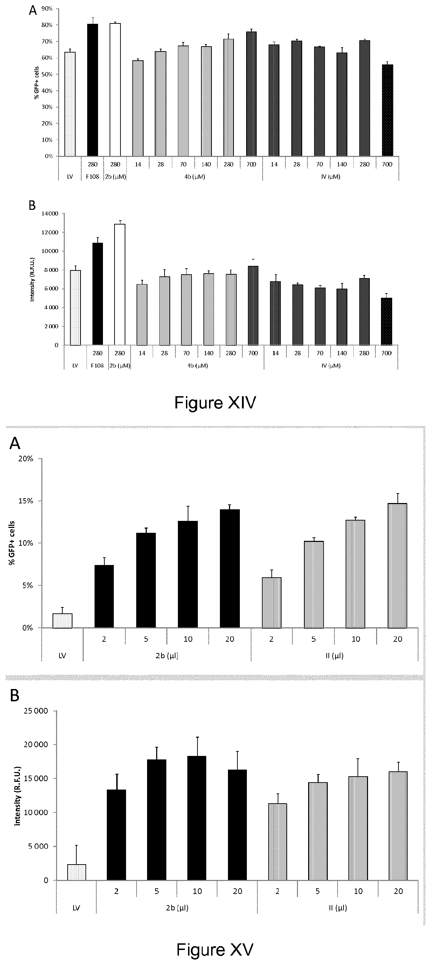

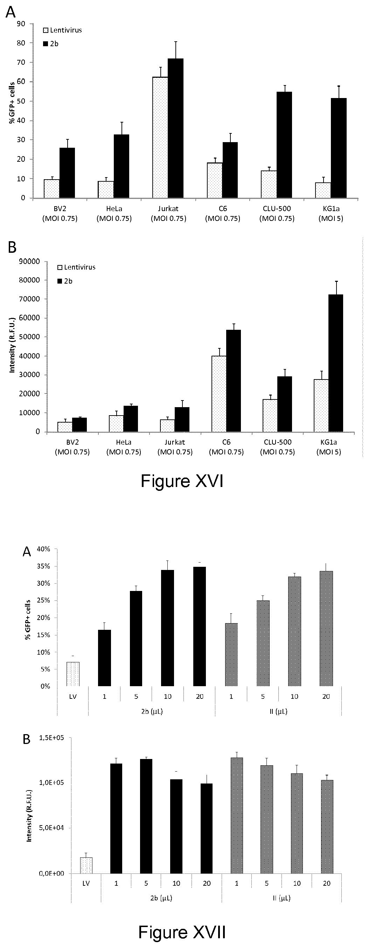

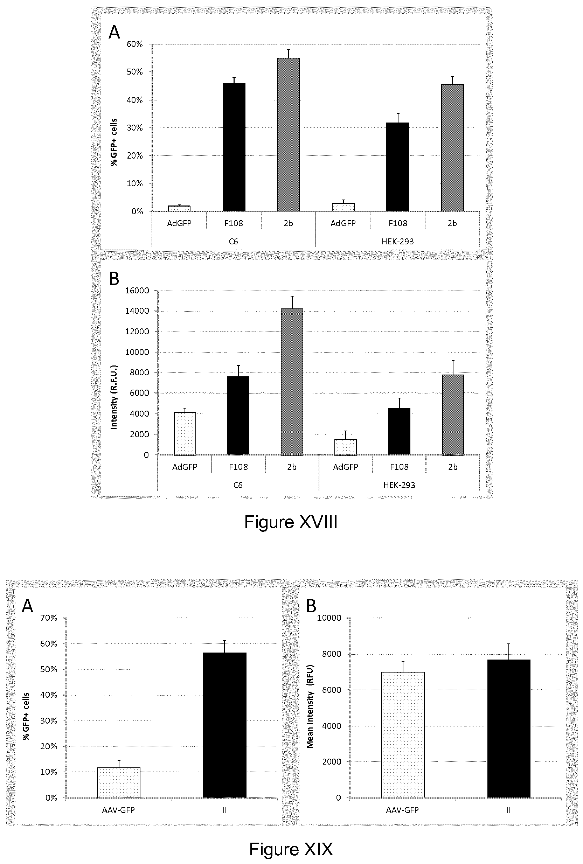

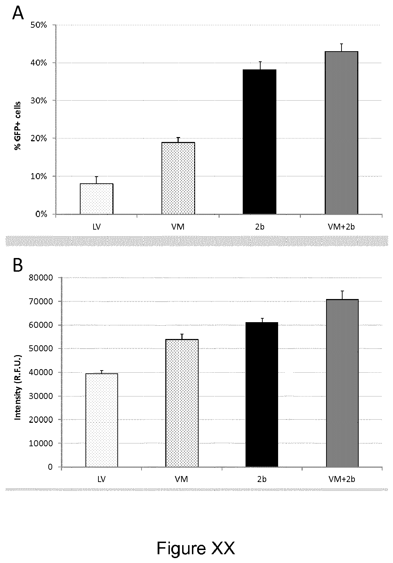

## Claims

1. Method for transducing a target cells with a viral vector using a cationic poloxamer.

2. Method according to claim 1, **characterized in that** said cationic poloxamers is used alone or formulated with nanoparticles.

3. Method according to claim 1 and 2, **characterized in that** said cationic poloxamers is used alone or in any composition comprising it.

4. Method according to anyone of claims 1 to 3, comprising the step of contacting said target cells with a viral vector and a cationic poloxamers.

5. Method according to claim 4, wherein said target cells are contacting with cationic poloxamer prior, at the same time or after contacting with viruses.

6. Method according to claim 4, wherein the cationic poloxamer and the viral particle are added at the same time onto the cells as mixture, or sequentially.

7. Method according to anyone of claims 1 to 6, **characterized in that** the contact between the virus, the cationic poloxamer and the cells ranges from 5 seconds to 3 months, preferentially comprised within 10 minutes to 2 weeks, more preferentially within 20 minutes to 1 week, even more preferentially comprised from 0.5h to 120h.

8. Method according to claim 1 to 7, wherein said cationic poloxamer is provided at a stock concentration of 0.01 to 500 mg/ml, preferentially from 0.05 to 300 mg/mL, more preferentially from 1 to 200 mg/mL, even more preferentially from 5 to 150 mg/mL.

9. Method according to anyone of claims 2 to 8, **characterized in that** when said cationic poloxamer is used formulated with nanoparticles, said nanoparticles are magnetic nanoparticles.

10. Method according to anyone of claims 2 or 9, **characterized in that** a further step of magnetic field is applied when the virus, the cells and the adjuvant have been put in contact for a time from 10 seconds to 96 h, preferentially from 1 minute to 48 h, more preferentially 5 min to 4 h.

11. Method of anyone of claims 1 to 10 comprising a further step of spinoculation or centrifugation prior to or after contacting said target cell with a virus and a cationic poloxamer.

12. Novel linear or branched cationic poloxamer according to formula I or formula II: 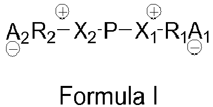 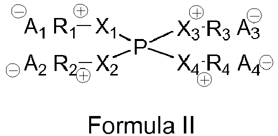 wherein
P is according to Formula III a or b: 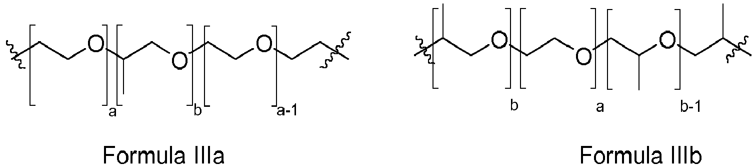 or
P is according to Formula IV or b: 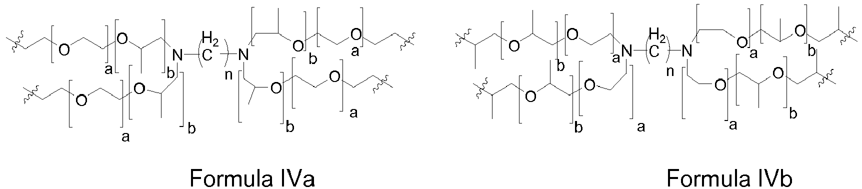
And wherein
- "***a***" represent the number of hydrophilic units repeated in the polymeric backbone P, and is an integer that ranges from 2 to 10000, preferentially, from 5 to 1000, more preferentially from 20 to 200;
- *"**a-1**"* is "***a***" described before in which 1 unit has been subtracted and is an integer that ranges from 2 to 10000, preferentially from 5 to 1000, more preferentially from 20 to 200;
- "***b***" is the number of hydrophobic unit repeated in the polymeric backbone P and is an integer that ranges from 2 to 1000, preferentially from 5 to 500, more preferentially from 15 to 80;
- *"**b-1**"* is the number "***b***" described before, in which 1 unit has been subtracted and is an integer that ranges from 2 to 1000, preferentially from 5 to 500, more preferentially from 15 to 80;
- **X₁**, **X₂, X₃ and X₄,** same or different, refer to heteroatoms, preferentially chosen from Nitrogen, Phosphorous, Silicon and Sulphur, and are covalently bonded to respectively **R₁, R₂, R₃ and R₄;**
- **n** is an integer comprised between 1 to 20 more preferentially between 2 to 6;
- **R₁, R₂, R₃ and R₄** are simultaneously or independently
• 1 to 6 hydrogen atoms, preferably 2 to 4.
• 1 to 8 heteroatoms, preferably 2 to 4, to be chosen between, nitrogen, phosphorous, sulphur and oxygen.
• 1 to 24, preferably 1 to 12, more preferentially 1 to 6, linear, branched and/or cyclic, saturated or unsaturated hydrocarbon group comprising from 1 to 24 carbon atoms, incorporating or not one or more heteroatoms such as oxygen, nitrogen, sulphur, phosphorous.
• 1 to 6, more preferentially 1 to 3 amino acid residues, natural or not.
• Any combination of these definitions
- **A₁, A₂, A₃ and A₄"** can represent the counter ions identical or different that can be chosen from one or several of the organic groups such as for example
∘ Halogen-based anions, such as for example iodide, bromide, chloride or fluoride;
∘ Organic groups bearing a negative charge, centred or not on a carbon atom such as for example methanesulfonate, trifluoromethanesulfonate, trifluoroacetate, acetate, formate, para-toluenesulfonate, carbonate, hydrogenocarbonate;
∘ Inorganic anions, such as for example sulphate, phosphate, nitrate, hydrogenosulphate, hydrogenophosphate;
∘ Inorganic, non-coordinating inorganic anions, such as tetrafluoroborate, hexafluorophosphate or perchlorate, carba-closo-dodecaborate;
∘ Boron-centered organic anions based on tetrakis[3,5-bis(trifluoromethyl)phenyl]borate backbone;
with the exception of F108 backbone, wherein a=132.7, b=50.3, a-1=131.7, and X1R1=X2R2=NH2; and F127 backbone, wherein a=100.22, b=65.2, a-1 =99.22, and X1R1=X2R2=NH2.

13. Novel linear or branched cationic poloxamer according to claim 12, **characterized in that** "**-X₁-R₁**", "**-X₂-R₂**", "**-X₃-R₃**" and "**-X₄-R₄**" is a
∘ Primary, secondary or tertiary amines cationic moiety. Amines derivates such as guanidines, hydrazines, guanidinium, hydrazinium can be preferred entities according to the invention;
∘ Organic quaternary phosphonium moieties such as for example substituted tri-*n-*butylphosphonium, substituted triphenylphosphonium, substituted triethylphosphonium;
∘ Quaternary ammonium salts based on a nitrogen atom covalently linked to 4 carbon moieties such as for example substituted quaternary trimethylammonium, substituted quaternary tri-*n*-butylammonium, substituted quaternary tri-*n-*octylammonium;
∘ Organic tertiary sulfonium salts based on a sulphur atom covalently linked to 3 carbon moieties such as for example substituted dimethyl sulfonium, substituted di-*n*-butylsulfonium, substituted di-*n*-octylsulfonium);
∘ Organic heterocycles bearing a net positive charge, delocalized or not on the cycle, incorporating at least 1 to 6 similar or different heteroatoms such as oxygen, nitrogen, sulphur, phosphorous and including at least one unsaturation providing them an aromatic character.
∘ Basic amino-acids residue as a source of cationic charges, such as for example residues of lysine, arginine, histidine, ornithine, tryptophane, natural or not;
∘ Natural non-polymeric polyamines, such as for example spermine, spermidine or thermospermine derivatives that are not of polymeric nature.

14. Cationic poloxamer according to claim 13, **characterized in that** said Organic heterocycles bearing a net positive charge are chosen from pyridine, and its cationic counterpart pyridinium; imidazole and its cationic counterpart imidazolium; triazole and its cationic counterpart triazolium; piperidine and its cationic counterpart piperidinium; morpholine and its cationic counterpart morpholinium.

15. Compounds according to claim 13 or 14, chosen from the compounds of formula:

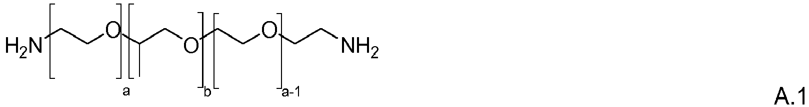

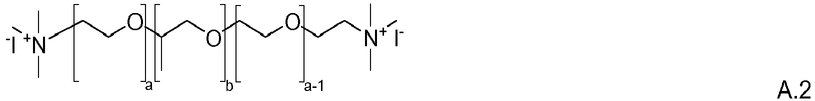

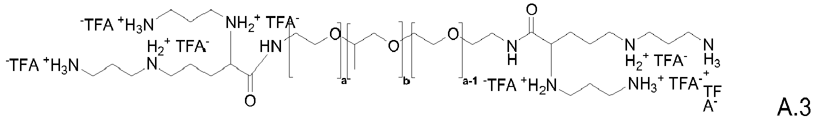

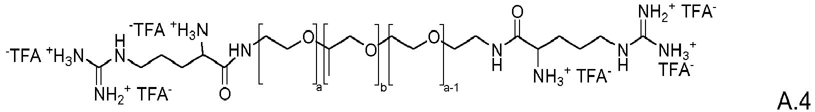

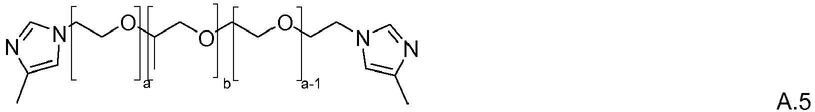

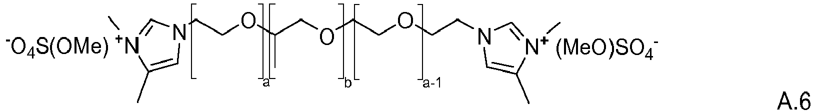

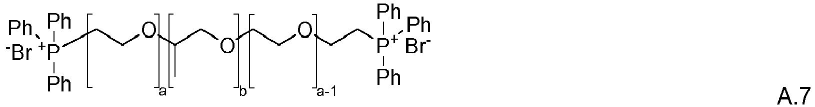

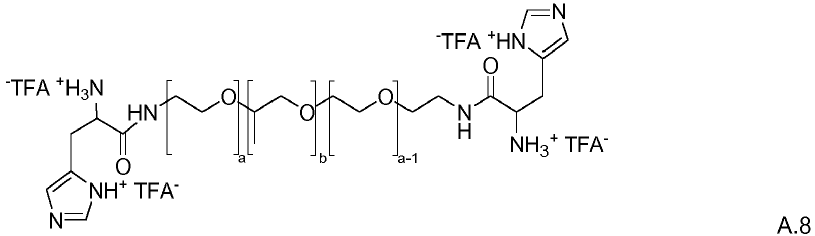

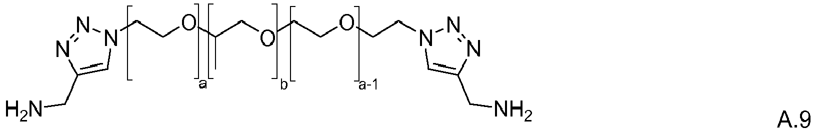

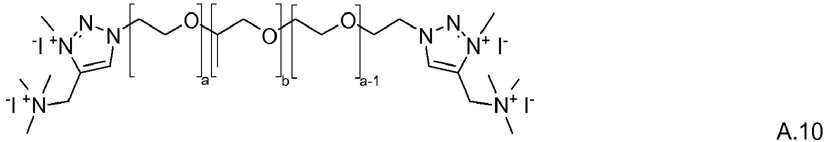

16. Composition, preferably therapeutic or cosmetic or for life sciences, comprising a compound of formula I or II as defined in anyone of claims 12 to 15.

17. A composition comprising a cationic poloxamer and a virus to transduce a target cell.

18. A kit comprising a cationic poloxamer alone or formulated as defined in any one of claims 1 to 15 further comprising a virus and optionally instructions of use.

19. A population of transduced cells obtained by the method of claim 1 to 11.

20. A method of treating a subject in need of a treatment with cell gene therapy, said method comprising administering to said subject an effective amount of the population of transduced cells of claim 19.
